(19) Europäisches Patentamt
European Patent Office
Office européen des brevets

(11) Publication number: 0 157 963 B1

(12) EUROPEAN PATENT SPECIFICATION

(45) Date of publication of patent specification: 21.06.89

(51) Int. Cl.[4]: C 07 D 327/02, C 07 D 411/00, C 07 D 417/04, A 61 K 31/41, A 61 K 31/39

(21) Application number: 84302097.5

(22) Date of filing: 28.03.84

# (54) 6H-dibenz (b,e)(1,4)oxathiepin compounds.

(43) Date of publication of application: 16.10.85 Bulletin 85/42

(45) Publication of the grant of the patent: 21.06.89 Bulletin 89/25

(84) Designated Contracting States: AT BE CH DE FR GB IT LI LU NL SE

(56) References cited: EP-A-0 105 692

(73) Proprietor: MERCK & CO. INC.
126, East Lincoln Avenue P.O. Box 2000
Rahway New Jersey 07065-0900 (US)

(73) Proprietor: MERCK FROSST CANADA INC.
16711 Trans-Canada Highway
Kirkland Quebec (CA)

(72) Inventor: Cragoe, Edward J., Jr.
2211 Oak Terrace Drive
Lansdale Pennsylvania 19446 (US)
Inventor: Rooney, Clarence S.
2902 Hickory Hill Drive
Worcester Pennsylvania 19490 (US)
Inventor: Rokach, Joshua
416 Canterbury Place Chomedey
Laval Quebec, H7W 4G9 (CA)

(74) Representative: Crampton, Keith John Allen et al
D YOUNG & CO 10 Staple Inn
London WC1V 7RD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 157 963 B1

Courier Press, Leamington Spa, England.

**Description**

This invention relates to dibenzoxathiepins. More particularly, the invention provides a novel group of 6H-dibenz[b,e][1,4]oxathiepins as defined below, as well as methods of preparing such compounds and their use in the treatment and control of allergic conditions such as asthma.

The present invention provides isomeric 6H-dibenz[b,e][1,4]oxathiepin compounds having either of the following structural formulae:

I 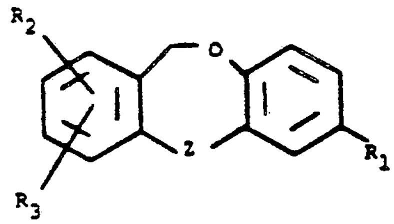 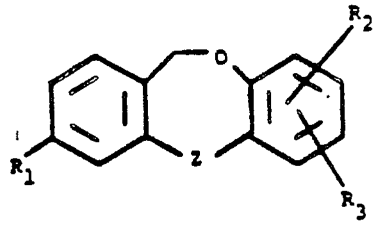 Ia

in which

Z is thia, sulfinyl, or sulfonyl;

each of $R_2$ and $R_3$, which are the same or different, is hydrogen, halogen, nitro, $C_{1-4}$ alkyl, amino, $C_{1-4}$ N-alkylamino, N,N-di($C_{1-4}$ alkyl)amino, $C_{1-4}$ alkanoyl, hydroxy, $C_{1-4}$ alkoxy, $C_{1-4}$ alkylthio, trifluoromethylthio, $C_{1-4}$ alkylsulfinyl, $C_{1-4}$ alkylsulfonyl, or trifluoromethyl;

$R_1$ is 5-tetrazolyl, 5-tetrazolylmethyl, 3-hydroxy-1,2,5-thiadiazol-4-yl, 4-hydroxy-$\Delta^3$-pyrrolin-3-yl-2,5-dione or

$$(CH_2)_n—\overset{O}{\overset{\|}{C}}—R_4$$

where n is 0 or an integer of from 1 to 4 and $R_4$ is N,N-di($C_{1-4}$ alkyl)amino-($C_{1-4}$ alkoxy), $C_{1-4}$ hydroxy alkoxy, carboxy-($C_{1-4}$ alkoxy), amino, $C_{1-4}$ N-alkylamino, N,N-di($C_{1-4}$ alkyl)amino, $C_{1-4}$ alkylsulfonylamino, carboxy($C_{1-4}$ alkyl)amino, carboxamido-($C_{1-4}$ alkyl)amino, 2-imino-3-methylthiazolidine, ($C_{1-4}$ acyloxy)-($C_{1-4}$ alkoxy) or (5-methyl-2-oxo-1,3-dioxolan-4-yl)methoxy;

and compounds that are pharmaceutically acceptable salts thereof.

The term "halogen" includes chlorine, bromine, iodine and fluorine; the $C_{1-4}$ alkyl, $C_{1-4}$ alkoxy and $C_{1-4}$ alkanoyl radicals are straight or branched, for example, methyl, ethyl, isopropyl, butyl, ethoxy, propoxy, isobutoxy, formyl, acetyl, propanoyl and butyryl.

It should be noted that European Patent Specification EP—A—0 105 692 discloses a class of 6H-dibenz[b,e][1,4]oxathiepin derivatives that can be used in the treatment and control of allergic conditions such as asthma. The compounds have the formula

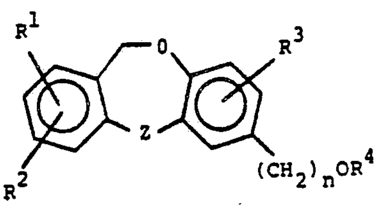 or 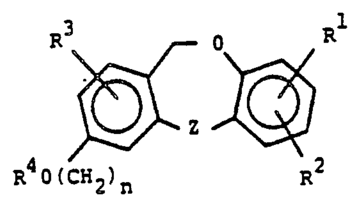

in which n is 1, 2, 3 or 4, Z is thia, sulfinyl or sulfonyl; each of $R^1$, $R^2$ and $R^3$ is H, halogen, $NH_2$, alkyl, alkylthio, alkylsulfinyl, alkylsulfonyl, $CF_3$, $CF_3S$, CN, $NO_2$, alkylamino, dialkylamino, alkoxy, alkanoyl, aralkyl, or hydroxyalkyl, or $R_1$ and $R_2$ together represent polymethylene, possibly oxo or hydroxy substituted; and $R_4$ is H, acyl, alkylaminoacyl, alkylcarboxy, alkylcarbamoyl, alkylcarbamoylacyl or acyloxyalkyl.

The present invention is based upon the discovery that oxathiepins of formulae I and Ia above markedly antagonize the actions of contractile prostaglandins such as $PGF_{2\alpha}$, $PGG_2$, $PGH_2$ and $TXA_2$. The use of the oxathiepins of this invention, which act as prostaglandin antagonists, and biosynthesis inhibitors, offers a new approach to therapy in a variety of allergic conditions such as allergic asthma where excessive contractile activity of prostaglandins and prostaglandin biosynthetic intermediates occur. It is well known, for example, that prostaglandins such as $PGF_{2\alpha}$, $PGG_2$, $TXA_2$ and $PGH_2$ are potent contractants of bronchial muscle and that human asthmatics are especially sensitive to the bronchial constricting action of $PGF_{2\alpha}$. The antagonizing action of the oxathiepins of this invention against the constricting actions of contractile prostaglandins has been confirming *in vitro* and *in vivo* using standard pharmacological techniques. It is contemplated, therefore, that the oxathiepins of this invention will be used in dosage unit form as the essential active ingredient in pharmaceutical formulations intended for the treatment and control of allergic conditions such as asthma in humans and warm blooded animals.

The novel oxathiepins of this invention, are conveniently prepared from the 2- (or 9-)cyano intermediates having the following structural formulae:

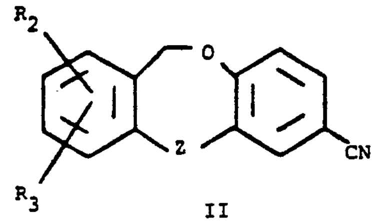

II

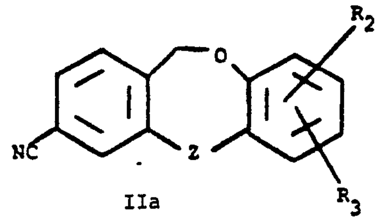

IIa

wherein $R_2$ and $R_3$ are as previously defined.

These 2-(or 9-)cyano-6H-dibenz[b,e][1,4]oxathiepin intermediates are themselves readily prepared from well known starting materials which are either available commercially or may be prepared by conventional techniques already fully described in the chemical literature.

Thus, 2-cyano-6H-dibenz[b,e][1,4]oxathiepin (II) may be prepared according to the following general reaction scheme:

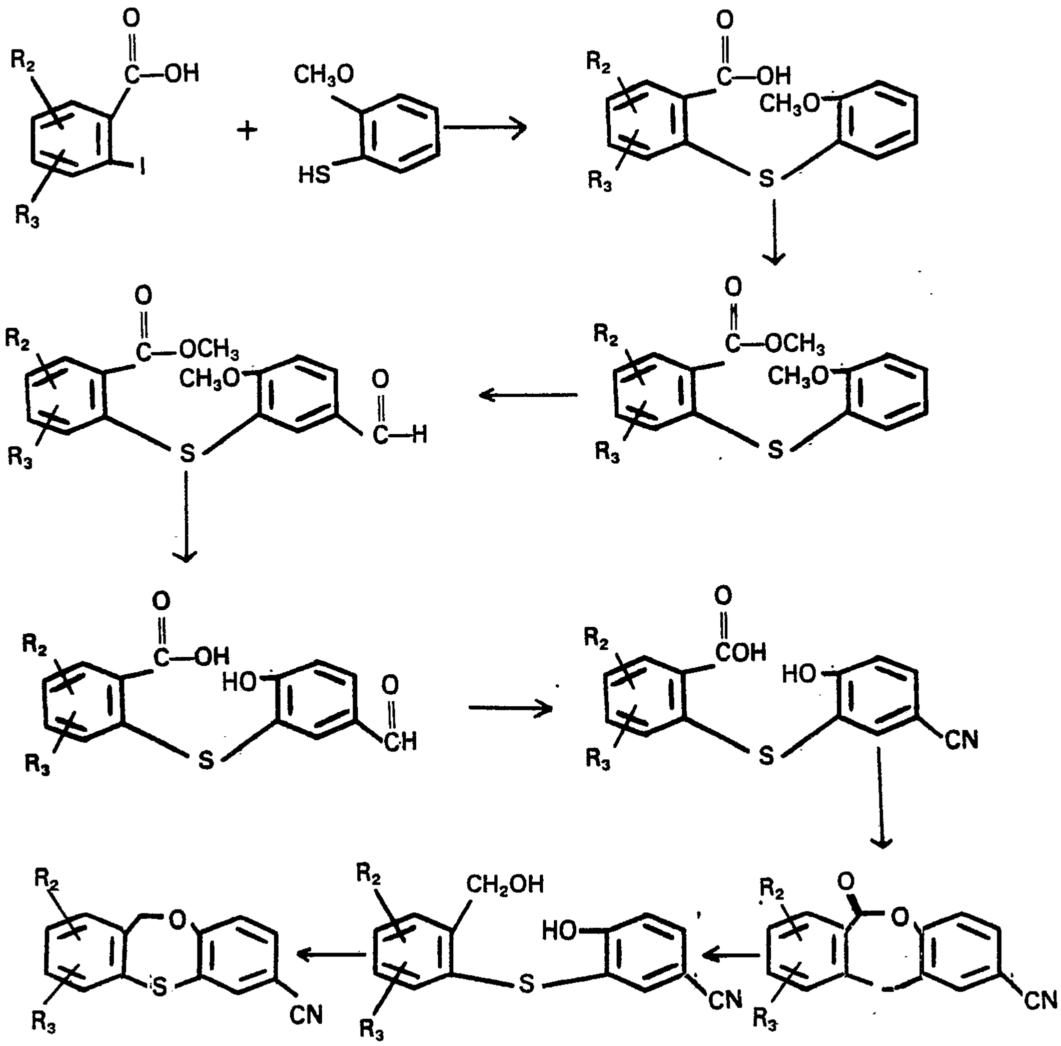

where $R_2$ and $R_3$ are as previously defined, by treating an appropriately substituted ($R_2$ and/or $R_3$) o-iodobenzoic acid with o-methoxythiophenol in the presence of copper powder and aqueous potassium hydroxide in order to obtain the corresponding 2-(o-methoxyphenylthio)benzoic acid. The reaction is carried out at reflux and usually requires 2 to 5 hours for completion. Upon recovery, the acid product may be converted into the corresponding lower alkanol ester by refluxing with a lower alkanol in the presence of a strong acid such as sulfuric acid, hydrochloric acid, trifluoroacetic acid and the like. The ester product then is treated with dichloromethyl methyl ether in the presence of titanium tetrachloride to form the corresponding 3-(o-carboloweralkoxyphenylthio)-4-methoxybenzaldehyde which then is demethylated with hydrogen bromide in glacial acetic acid to form the corresponding 3-(o-carboxyphenylthio)-4-hydroxybenzaldehyde. The aldehyde so produced then is treated with hydroxylamine hydrochloride in the presence of sodium formate and formic acid to form the corresponding 3-(o-carboxyphenylthio)-4-hydroxybenzonitrile which is treated with dicyclohexylcarbodiimide (DCC) to form the corresponding 2-cyano-6H-6-oxo-dibenz[b,e][1,4]oxathiepin. The 2-cyano-oxathiepin product then is treated with an alkali

metal borohydride to form the corresponding 3-(o-hydroxymethylphenylthio)-4-hydroxybenzonitrile which is reacted with dicyclohexylcarbodiimide to form the desired 2-cyano-6H-dibenz[b,e][1,4]oxathiepin of formula II.

9-Cyano-6H-dibenz[b,e][1,4]oxathiepin (IIa) may be prepared according to the following general reaction scheme:

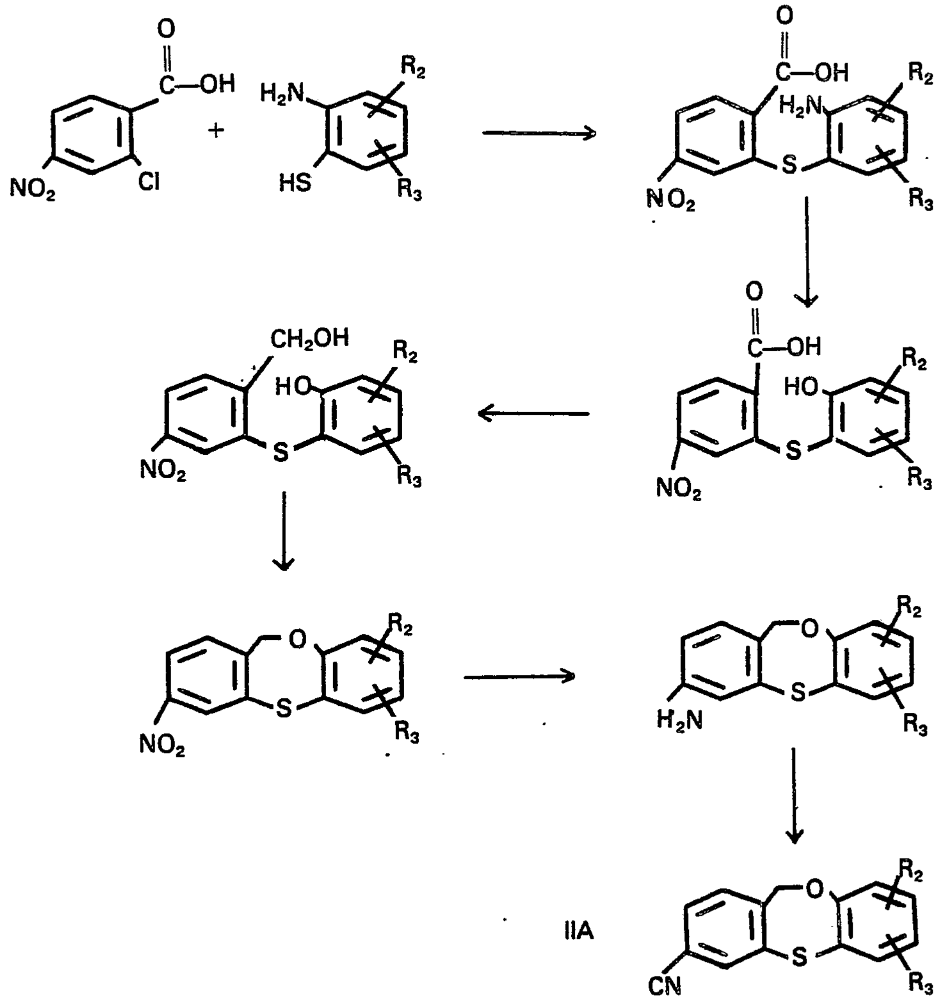

IIA

wherein $R_2$ and $R_3$ are as previously defined, by treating an appropriately substituted ($R_2$ and/or $R_3$) o-aminothiophenol with 2-chloro-4-nitrobenzoic acid in the presence of cuprous oxide to form the corresponding 2-(o-aminophenylthio)-4-nitrobenzoic acid which then is suspended in water, acidified with sulfuric acid, treated with sodium nitrite followed by sodium fluoroborate to obtain the diazonium fluoroborate which is heated in 50% sulfuric acid to obtain the corresponding 2-(o-hydroxyphenylthio)-4-nitrobenzoic acid. The acid product is treated with borane in tetrahydrofuran to form the corresponding 2-(o-hydroxyphenylthio)-4-nitrobenzyl alcohol which is cyclized in the presence of dicyclohexylcarbodiimide (DCC) to form the corresponding 9-nitro-6H-dibenz[b,e][1,4]oxathiepin. The nitro group then is reduced to the 9-amino compound with stannous chloride and the amine is treated with sodium nitrite followed by a mixture of cuprous cyanide and potassium cyanide to obtain the desired 9-cyano-6H-dibenz[b,e][1,4]oxathiepin of formula IIa.

The oxathiepins in which the substituent at the 2-(or 9-) position is carboxy are prepared by refluxing the 2-(or 9-)cyano intermediates of formula II or IIa in a mixture of ethanol and aqueous sodium hydroxide. The reaction usually requires from 2 to 6 hours for completion and the desired 6H-dibenz[b,e][1,4]oxathiepin-2-(or 9-)carboxylic acid of formula III or IIIa is recovered from the reaction mixture upon acidification.

II 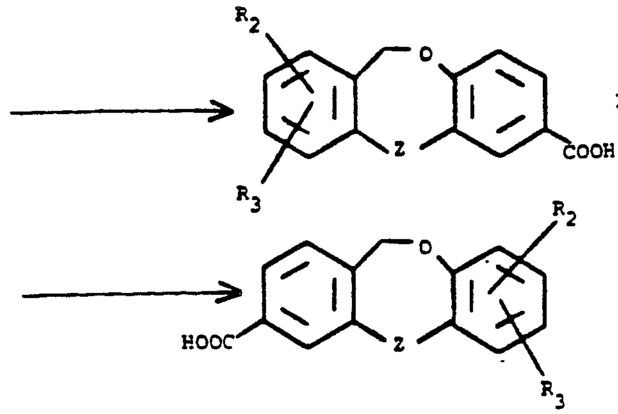 III

IIa IIIa

The novel oxathiepins of this invention wherein the substituent at the 2-(or 9-)position is 5-tetrazolyl also are prepared from the 2-(or 9-)cyano intermediates of formula II or IIa. The nitrile is heated in a mixture of sodium azide and ammonium chloride in a suitable organic solvent such as dimethylformamide. Conveniently, the reaction is carried out at reflux and usually requires 4 to 12 hours for completion. After dilution with excess sodium carbonate and extraction with ethyl acetate, the aqueous phase is acidified to obtain the desired 2-(or 9-)(1H-tetrazol-5-yl)-6H-dibenz[b,e][1,4]oxathiepin of formula IV or IVa.

II 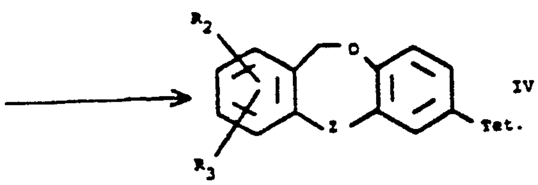 IV

IIa 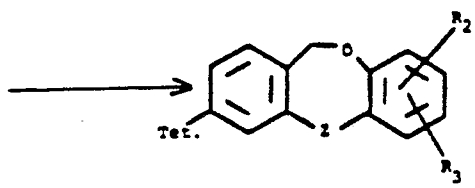 IVa

Formation of the 11-oxide or the 11,11-dioxide groups (e.g., preparation of the sulfinyl or sulfonyl compounds of the instant invention) conveniently is achieved by controlled oxidation techniques. Thus, for example, the carboxylic acid derivatives of formula III or IIIa may be oxidized with hydrogen peroxide in the presence of an acidic solvent such as acetic acid or with organic peroxides such as peroxy acids, for example, m-chloroperbenzoic acid and the like, in a stepwise fashion to form the corresponding sulfinyl derivative, formula V or Va, and sulfonyl derivative, formula VI or VIa, by controlling the molar ratio of oxidant to reductant. The molar ratio determines the oxidation level of the sulfur in the product. A 1:1 molar ratio, for example, results largely in the production of the sulfinyl derivative whereas a 2 to 3 molar excess of oxidant results in a yield predominantly comprising the sulfonyl derivatives.

IIIa 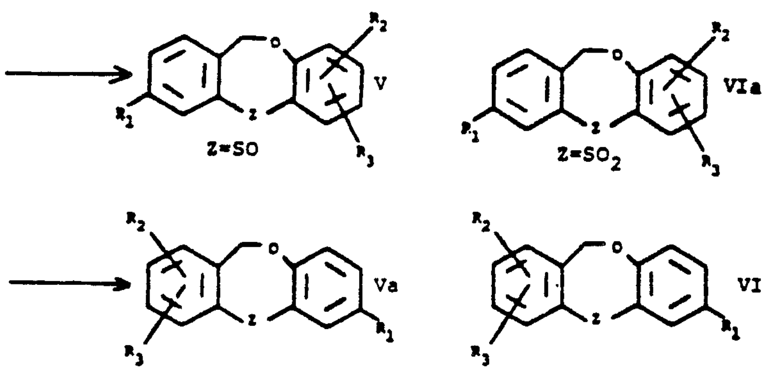

V Z=SO

VIa Z=$SO_2$

III

Va

VI

The oxidation technique described above is generally applicable to preparation of any of the sulfinyl or sulfonyl compounds of this invention from the corresponding oxathiepin. Thus, for example, the 5-tetrazolyl compounds of formula IV or IVa may be subjected to controlled oxidation in order to obtain the corresponding sulfinyl and sulfonyl compounds.

Alternatively, the 2-(or 9-)cyano intermediates of formula II or IIa may be oxidized as described above to produce the corresponding sulfinyl or sulfonyl intermediate. These intermediates then may be converted to the corresponding 2-(or 9-)carboxylic acid or 5-tetrazolyl compounds by the methods already described.

In addition to their therapeutic properties as noted above, the 2-(or 9-)carboxylic acid derivatives of this invention serve as valuable intermediates in the preparation of other variously substituted and therapeutically useful 6H-dibenz[b,e][1,4]oxathiepins of formula I and Ia. Thus, the 2-(or 9-)carboxylic acid of formula III or IIIa may be converted readily into the corresponding acid halide, preferably the acid chloride, by treating the carboxylic acid with a thionyl halide, preferably thionyl chloride. The resulting 2-(or 9-)halo-carbonyl-6H-dibenz[b,e][1,4]oxathiepin (i.e., the 2-(or 9-)chlorocarbonyl compounds of formula VII or VIIa) then may be treated with various well-known reagents to form desired ester and amide derivatives.

III
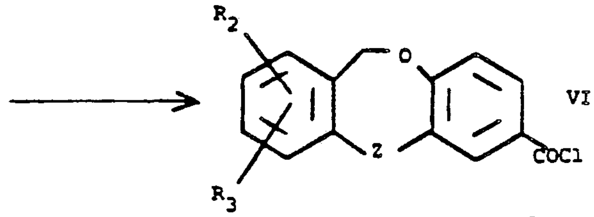
VII

IIIa
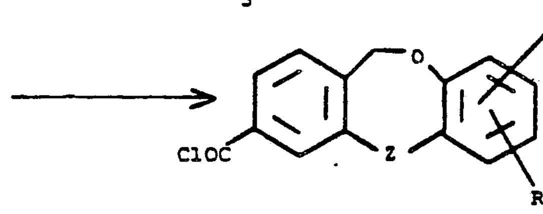
VIIa

Thus, for example, the chlorocarbonyl compounds of formula VII and VIIa may be treated:

(a) with a loweralkanol such as, for example, methanol, ethanol, 2-propanol, butanol and 2-butanol, to form the corresponding (R=) loweralkyl esters, VIII and VIIIa:

VII
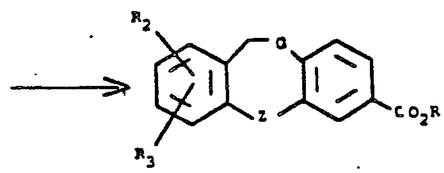
VIII

VIIa
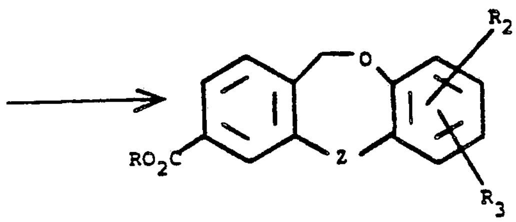
VIIIa

(b) with ammonia to form the corresponding carboxamides, IX and IXa:

VII
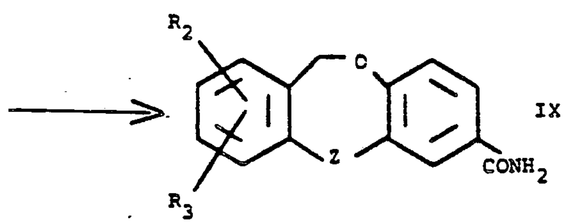
IX

VIIa
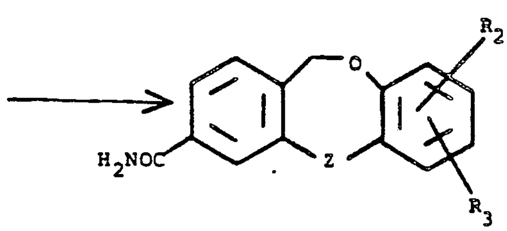
IXa

(c) with an N-loweralkylamine such as, for example, methylamine, ethylamine, propylamine, isopropylamine and butylamine, or an N,N-diloweralkylamine such as, for example, dimethylamine,

diethylamine, dipropylamine and dibutylamine, to form the corresponding ($R_1$=) N-loweralkylcarboxamide X or Xa, or N,N-diloweralkylcarboxamide, XI or XIa:

VII
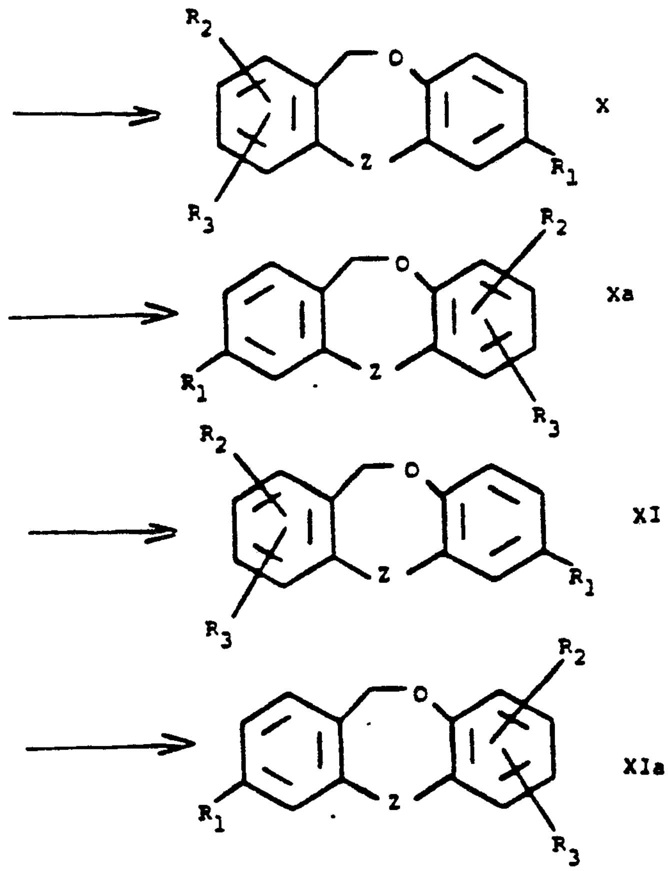
X

VIIa

Xa

VII

XI

VIIa

XIa

(d) with a loweralkylsulphonamide such as, for example, methanesulphonamide, ethanesulphonamide, propanesulphonamide and butanesulphonamide, to form the corresponding ($R_1$=) N-loweralkylsulfonylcarboxamide, XII or XIIa:

VII
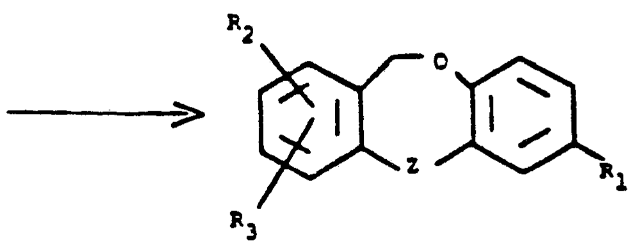
XII

VIIa
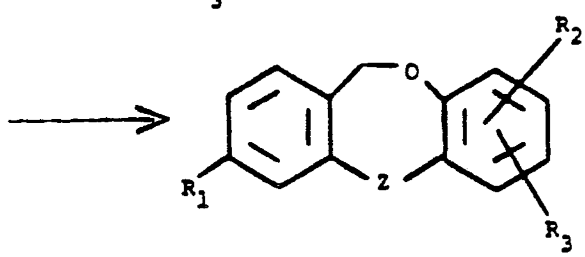
XIIa

(e) with 2-imino-3-methylthiazolidine to form the corresponding ($R_1$=) (3-methyl-2-thiazolidinylidene) carboxamide, XIII and XIIIa:

VII
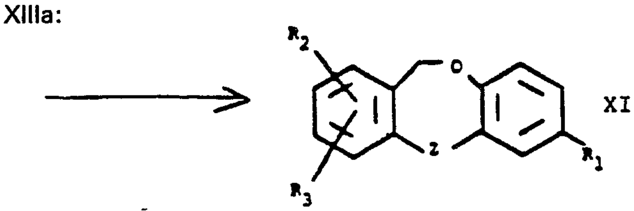
XIII

VIIa 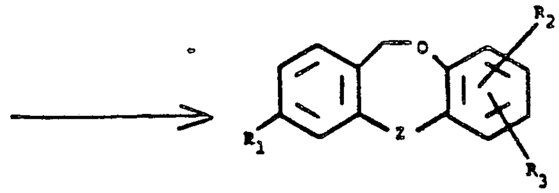 XIIIa

(f) with a loweralkyldiol such as, for example ethylene glycol, trimethylene glycol and 1,4-butanediol, to form the corresponding ($R_1$=) hydroxyloweralkylester, XIV and XIVa:

VII 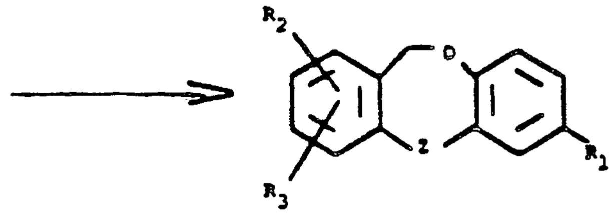 XIV

VIIa 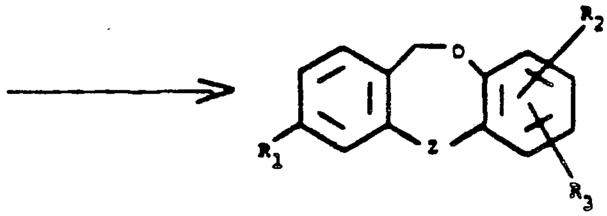 XIVa

(g) with an N,N-diloweralkylaminoloweralkanol such as, for example, N,N-dimethylethanolamine, N,N-diethylethanolamine, 3-N,N-dimethylaminopropan-1-ol and 4-N,N-diethylaminobutan-1-ol, to form the corresponding ($R_1$=) N,N-diloweralkylaminoloweralkyl ester, XV and XVa:

VII 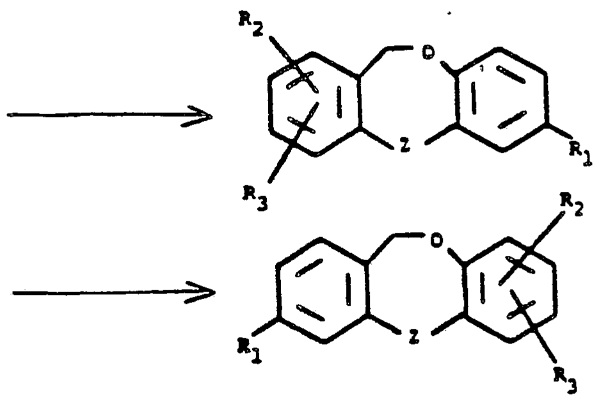 XV

VIIa XVa

(h) with an amino acid such as, for example, glycine, alanine and valine, to form the corresponding ($R_1$=) N-carboxyloweralkylcarboxamide, XVI and XVIa:

VII 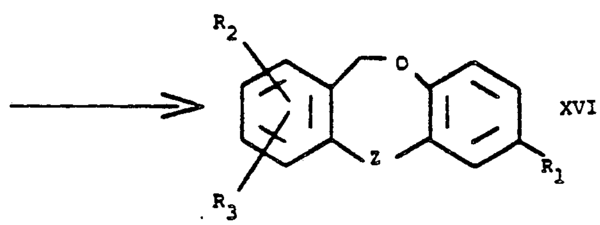 XVI

VIIa 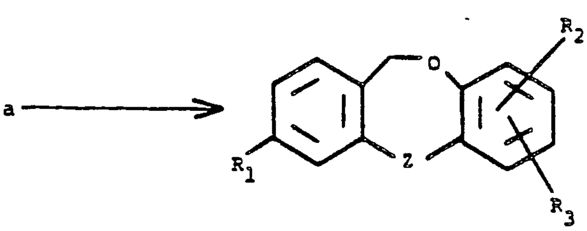 XVIa

(i) with an alkali metal salt of a hydroxyloweralkanoic acid such as, for example, hydroxyacetic acid, 3-hydroxybutyric acid and β-hydroxypropionic acid, to form the corresponding ($R_1$=) carboxyloweralkyl ester, XVII and XVIIa:

VII 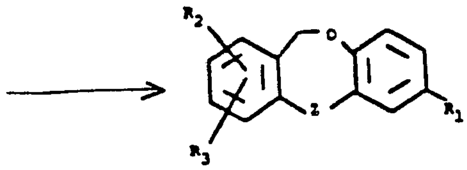 XVII

VIIa 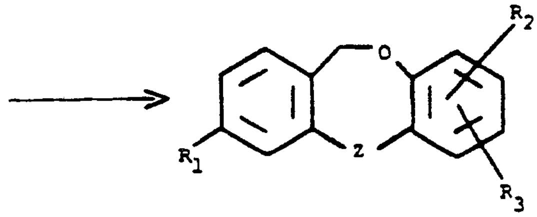 XVIIa

Where the corresponding sulfinyl or sulfonyl derivatives are desired, the corresponding 11-oxide or 11,11-dioxide 2-(or 9-)carboxylic acid, e.g. a compound of formula V, Va, VI or VIa, may be substituted for starting material III or IIIa in the foregoing reaction sequence. Alternatively, it will be clear to those skilled in the art that the product esters and amides obtained in the foregoing reaction sequence may be oxidized by the techniques already described to obtain the corresponding sulfinyl or sulfonyl derivatives.

Those oxathiepins of this invention wherein the substituent at the 2-(or 9-)position is 3-hydroxy-1,2,5-thiadiazol-4-yl are prepared by refluxing the 2-(or 9-)cyano intermediate in formic acid in the presence of Raney nickel alloy for 1 to 2 hours in order to obtain the corresponding 6H-dibenz[b,e][1,4]oxathiepin-2-(or 9-)carboxaldehyde. The aldehyde product then is converted into the corresponding 2-(or 9-)2-aminoacetonitrile by treatment with sodium cyanide in an alcoholic solvent saturated with ammonia and in the presence of ammonium chloride and ammonium hydroxide. The reaction usually is conducted at room temperature and requires from 8 to 16 hours to completion. The aminoacetonitrile so produced is treated with concentrated hydrochloric acid at room temperature for 20 to 45 minutes in order to obtain the corresponding 2-(or 9-)(2-aminoacetamide) which then is treated with sulfur monochloride in dimethylformamide to obtain the desired ($R_1$=) 2-(or 9-)(3-hydroxy-1,2,5-thiadiazol-4-yl) 6H-dibenz[b,e][1,4]oxathiepin of formula XVIII, XVIIIa. This reaction sequence is illustrated in the following diagram, it being understood that position of the 3-hydroxy-1,2,5-thiadiazol-4-yl substituent in the final product depends upon the selection of the appropriate 2-(or 9-)cyano starting material.

II or IIa

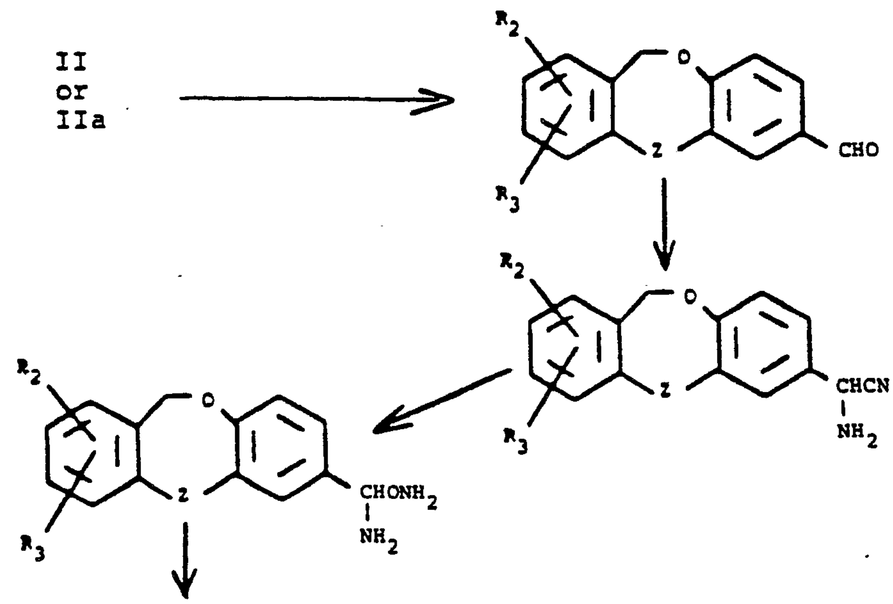

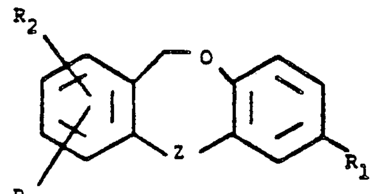

XVIII

OR

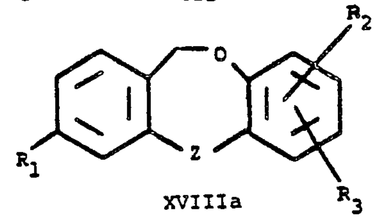

XVIIIa

The novel oxathiepins of this invention where the $R_1$ substituent at the 2-(or 9-)position is 4-hydroxy-$\Delta^3$-pyrroline-3-yl-2,5-dione are prepared from the appropriately substituted 2-(or 9-)carboxylic acid by reducing the acid to the corresponding alcohol with borane in tetrahydrofuran. The reaction conveniently is carried out at room temperature under an inert atmosphere and usually requires 2 to 4 hours for completion. The alcohol then is brominated with phosphorous tribromide and the bromomethyl so produced is treated with sodium cyanide to form the corresponding 2-(or 9-)cyanomethyl derivative. These reactions may be carried out at room temperature and usually require from 1 to 3 hours for completion.

The cyanomethyl intermediate then is hydrolyzed to the corresponding acetic acid which is treated with thionyl chloride followed by ammonia to form the corresponding 2-(or 9-)acetamide derivative by techniques already described. The acetamide then is treated with diethyl oxalate in dimethylformamide in the presence of potassium *t*-butoxide to form the desired ($R_1$=) 2-(or 9-)(4-hydroxy-$\Delta^3$-pyrrolin-3-yl-2,5-dione)-6H-dibenz[b,e][1,4]oxathiepin of formula XIX and XIXa.

This reaction sequence is illustrated in the diagram below, it again being understood that the position of the hydroxypyrrolinedione in the final product depends upon the selection of the 2-(or 9-)carboxylic acid starting material.

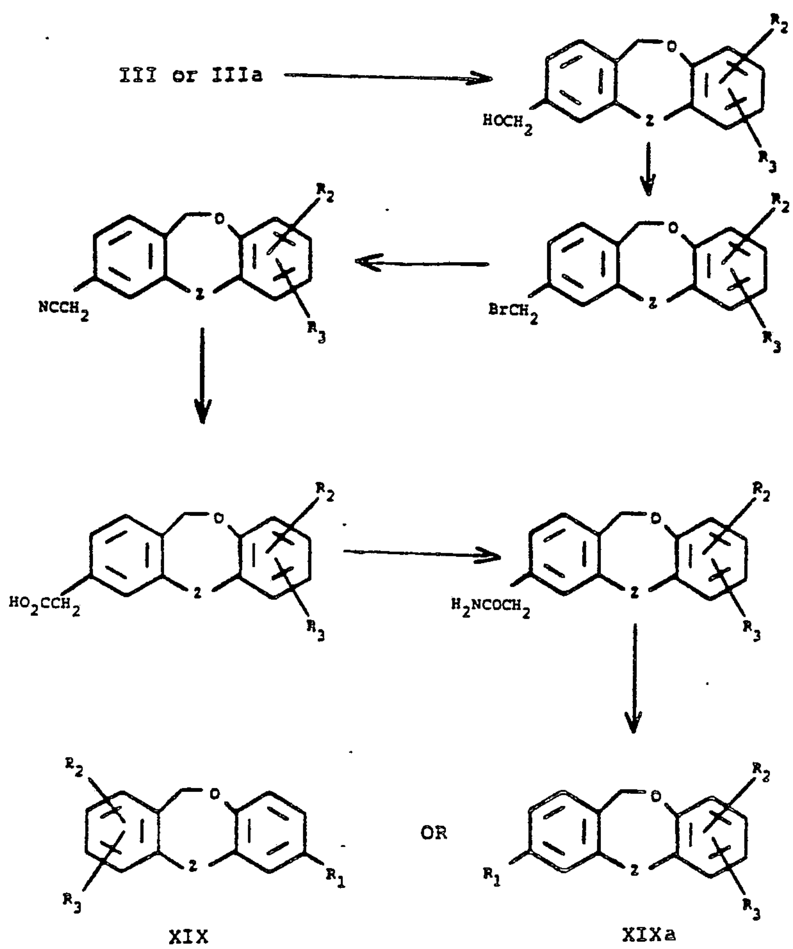


OR

XIX XIXa

Where corresponding sulfinyl or sulfonyl derivatives are desired, the products of both reaction schemes described above may be oxidized by the techniques already described.

It will be noted that the reaction sequence described above affords not only oxathiepins of this invention wherein the substituent at the 2-(or 9-)position is hydroxy-$\Delta^3$-pyrroline-3-yl-2,5-dione, but, in Steps A—D, lead also to the preparation of those oxathiepins in which the substituent at the 2-(or 9-) position is a loweralkanoic acid (i.e., compounds of formula I and Ia, wherein $R_1$ is

$$-(CH_2)_n-\overset{O}{\overset{\|}{C}}R_4,$$

n is an integer between 1 and 4 and $R_4$ is hydroxy). Thus, Steps A—D, as described above, starting with the appropriately substituted 2-(or 9-)carboxylic acid, through reduction, bromination, cyanization and oxidation afford the corresponding 2-(or 9-)acetic acid derivative. Quite obviously, the described reduction, bromination, cyanization and oxidation sequence can be repeated, employing the 2-(or 9-)acetic acid derivative as starting material, in order to obtain the corresponding propionic acid derivative which, in turn, can be employed as starting material for preparing the corresponding butyric acid derivative. In this manner, any desired 2-(or 9-)loweralkanoic acid derivative is readily prepared. Corresponding sulfinyl or sulfonyl derivatives are prepared by the oxidation techniques previously described.

The 2-(or 9-) cyano loweralkyl intermediates obtained from Steps A—C in the reaction sequence described above also serve as intermediates in the preparation of other therapeutically active oxathiepins of formula I or Ia. Thus, for example, an appropriately substituted 2-(or 9-) cyanomethyl-6H-dibenz[b,e][1,4]oxathiepin may be treated with sodium azide and ammonium chloride by techniques previously described to form the corresponding 2-(or 9-)(1H-tetrazol-5-yl-methyl)-6H-dibenz[b,e][1,4]oxathiepin and the product, if desired, can be oxidized to form the corresponding sulfinyl or sulfonyl derivative.

The halogen substituted 6H-dibenz[b,e][1,4]thiepin derivatives ($R_2$ or $R_3$=F, Cl, Br or I) are prepared as shown in the following reaction sequence which illustrates the preparation of 8-(or 9-)fluoro-6H-dibenz-[b,e][1,4]oxathiepin-2-carboxylic acid of formula XX and XXa and the corresponding -11,11-dioxide XXI and XXIa.

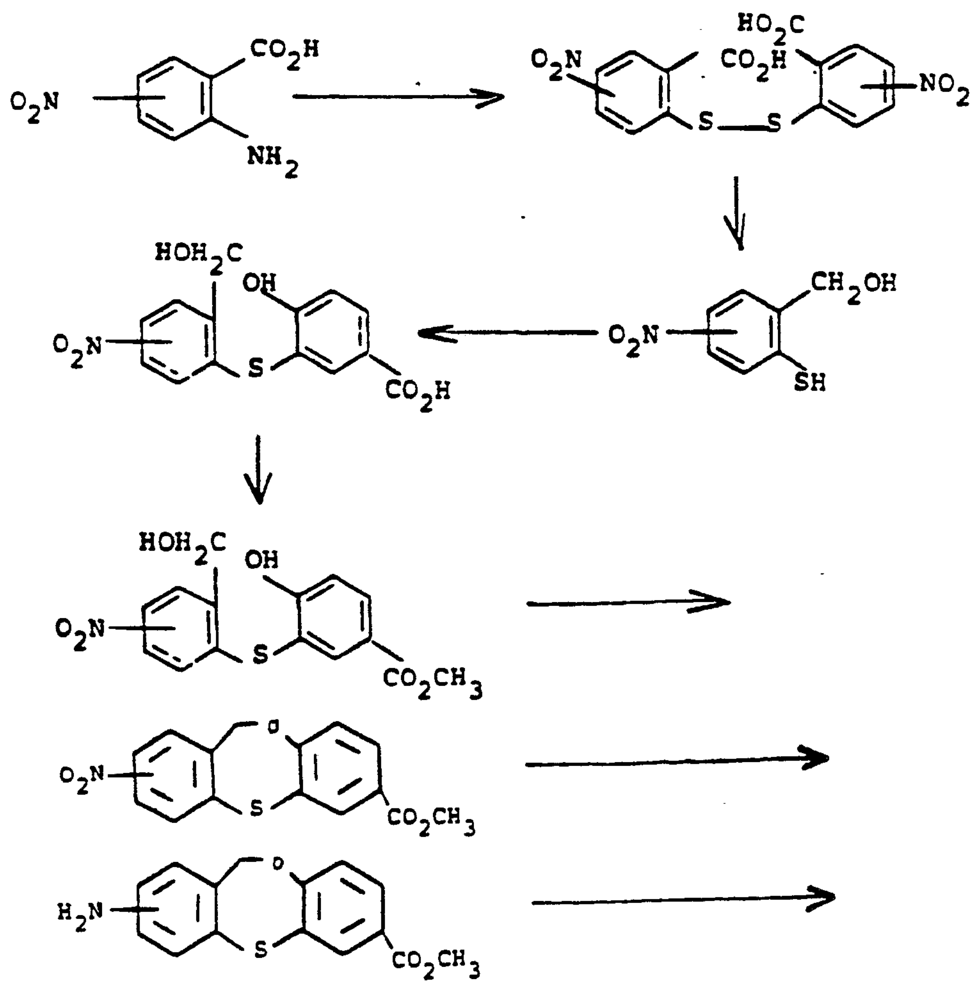

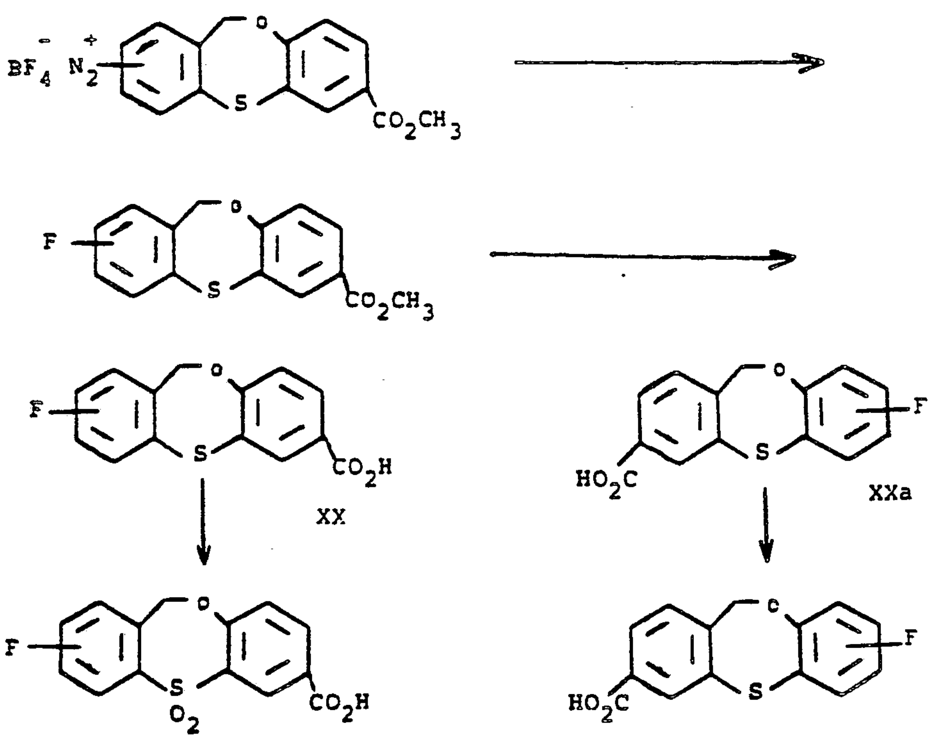

XX

XXa

XXI

XXIa

2-Amino-5-(or 4-) nitrobenzoic acid is converted into the disulfide carboxylic acid by reaction with (1) sodium nitrite and sulfuric acid; (2) potassium xanthogenate; and (3) sodium carbonate/water.

The disulfide is converted to 2-mercapto-5-(or 4-)nitrobenzyl alcohol by reductive cleavage using diborane.

Reaction of the mercaptonitrobenzyl alcohol with 4-hydroxy-3-iodo-benzoic acid in the presence of N-methyl-2-pyrrolidinone affords 3-(2-hydroxymethyl-4(or 5)-nitrophenylthio)-4-hydroxybenzoic acid.

Esterification of the benzoic acid is accomplished with methanol and sulfuric acid. Ring closure of the diol using diethyl azodicarboxylate and triphenylphosphine affords methyl 8-(or 9-)nitro-6H-dibenz[b,e]-[1,4]oxathiepin-2-carboxylate.

The 8-(or 9-) nitro group is then reduced using stannous chloride to afford the 8-(or 9-) amino compound. Reaction of the 8-(or 9-) amino compound with sodium nitrite and hydrogen tetrafluoroborate affords the diazonium tetrafluoroborate salt. Heating this salt affords the 8-(or 9-) fluoro compound.

Hydrolysis of the methyl 8-(or 9-) fluoro-6H-dibenz[b,e][1,4]oxathiepin-2-carboxylate with (1) sodium hydroxide/water; and (2) hydrochloric acid affords the 8- (or 9-) fluoro-6H-dibenz[b,e][1,4]oxathiepin-2-carboxylic acid. Treatment of the carboxylic acid with hydrogen peroxide/acetic acid yields the compound 8- (or 9-) fluoro-6H-dibenz[b,e][1,4]oxathiepin-2-carboxylic acid-11,11-dioxide.

As noted above, pharmaceutically acceptable salts of the novel oxathiepins also are included with the scope of this invention. The term, pharmaceutically acceptable salts, is intended to include salts derived from pharmaceutically acceptable non-toxic acids and bases such as, for example, ammonium salts, alkali metal salts such as sodium and potassium salts, alkaline earth metal salts such as magnesium and calcium salts, salts of organic bases such as amine salts derived from mono-, di- and triloweralkyl or hydroxy-loweralkanyl amines such as trimethylamine, dimethylamine and triethanolamine, salts derived from heterocyclic amines such as piperidine, 1-methylpiperazine, piperazine and morpholine, and salts derived from pharmaceutically acceptable acids such as hydrochloric acid, sulfuric acid, tartaric acid and propionic acid.

It will be noted that many of the carboxylic acid derivatives encompassed by formulae I and Ia will serve as pro-drugs of the active carboxylate form *in vivo.* Several of these derivatives also possess bioactivity in their own right. For a general discussion of pro-drugs see A. A. Sinkula, *Ann. Rpts. Med. Chem. 10* 306—316 (1975), wherein the utility of pro-drug forms, for example esters, amides and amino acid amide derivatives of carboxylic acids are shown to provide useful means of administering the drug.

The oxathiepins of formula I and Ia are useful in the treatment and prophylaxis of human or warm-blooded animal diseases conditions where excessive undesirable contractile activity of prostaglandins, such as $PGF_{2\alpha}$, or prostaglandin biosynthetic intermediates contribute. In particular, they are of value in the treatment and control of allergic conditions such as asthma.

The magnitude of a prophylactic or therapeutic dose of compound of formula I and Ia will, of course, vary with the nature and the severity of the condition to be treated and with the particular compound of

formula I and Ia and its route of administration. In general, the dose range lies within the range of 0.2 mg to 100 mg per kg body weight per day.

The pharmaceutical compositions of the present invention comprise a compound of formula I and Ia as an active ingredient, and may also contain pharmaceutically acceptable carrier and optionally other therapeutic ingredients. The compositions include compositions suitable for oral, rectal, ophthalmic, pulmonary, nasal, dermal, topical or parenteral (including subcutaneous, intramuscular and intravenous) administration, although the most suitable route in any given case will depend on the nature and severity of the condition being treated and on the nature of the active ingredient. They may be conveniently presented in unit dosage form and prepared by any of the methods well known in the art of pharmacy.

For use where a composition for intravenous administration is employed, a suitable dosage range is from 0.2 to 10 mg (preferably 1 to 8 mg) of a compound of formula I and Ia per kg of body weight per day and in the case where an oral composition is employed a suitable dosage range is about, e.g., 1 to 50 mg of a compound of formula I and Ia per kg of body weight per day, preferably from 5 to 40 mg/kg.

Pharmaceutical compositions of the present invention suitable for oral administration and by inhalation in the case of asthma therapy may be presented as discrete units such as capsules, cachets or tablets each containing a predetermined amount of the active ingredient; as a powder or granules; or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or moulding, optionally with one or more accessory ingredients. Compressed tablets may be prepared by compressing in a suitable machine, the active ingredient in a free-flowing form such as powder or granules, optionally mixed with a binder, lubricant, inert diluent, lubricating, surface-active or dispersing agent. Moulded tablets may be made by moulding in a suitable machine, a mixture of powdered compound moistened with an inert liquid diluent. Desirably, each tablet contains from 50 mg to 500 mg of the active ingredient and each cachet or capsule contains from 50 mg to 500 mg of the active ingredient.

The best mode contemplated by applicants for carrying out their invention is illustrated in the following working examples. No limitation, however, is intended except as set forth in the appended claims.

## Example 1

### 9-(1H-Tetrazol-5-yl)-6H-dibenz[b,e][1,4]oxathiepin-11,11-dioxide

Step A: 2-(o-Aminophenylthio)-4-nitrobenzoic acid

Heat a mixture of 466 g (3.72 moles) of 2-aminothiophenol, 250 g (1.24 moles) of 2-chloro-4-nitrobenzoic acid, 1.25 litres of quinoline, 192 g (1.34 moles) of cuprous oxide and 125 ml of pyridine in an oil bath at 160—170°C with mechanical stirring for 90 minutes. Cool the mixture to room temperature and add 1.87 litres of concentrated hydrochloric acid followed by 625 ml of water. Separate the precipitate and wash well with water. Extract the washed precipitate into boiling methanol and filter. Treat the hot filtrate with charcoil, filter and strip to dryness. Dissolve the residue in aqueous sodium hydroxide, filter and treat with charcoal. Acidify the filtrate and separate the precipitate. (Yield: 38 g).

*Additional Crop:* Take up the insoluble residue from the basic extraction into water, filter through Celite and acidify. Separate the precipitate and dissolve in ethyl acetate. Treat with charcoal, filter and strip to dryness. (Yield: 23 g).

Step B: 2-(o-Hydroxyphenylthio)-4-nitrobenzoic acid

Suspend 10.15 g (35 mmoles) of the amino acid of Step A in 75 ml of water and add 4 ml concentrated sulfuric acid (7.36 g, 75 mmoles, 140 meq.). Cool the mixture in an ice-bath and add 3.657 g (53 mmoles) of sodium nitrite in portions at 0—5°C. Stir the suspension in the cold for 20 minutes. Add 10 g (91 mmoles) of sodium fluoroborate and stir for an additional 20 minutes. Separate the precipitated crude diazonium fluoroborate, suspend the precipitate in 250 ml of 50% sulfuric acid and heat in an oil bath at 90—100°C for 45 minutes. Cool the mixture and separate the precipitate. (Yield: 7.76 g).

Step C: 2-(o-Hydroxyphenylthio)-4-nitrobenzyl alcohol

Dissolve 42 g (0.144 mole) of the acid of Step B in 575 ml of tetrahydrofuran and add dropwise 275 ml of borane (0.275 mole $BH_3$) in tetrahydrofuran under a nitrogen atmosphere at room temperature. Stir at room temperature overnight. Slowly add excess water and concentrate to remove the tetrahydrofuran. Extract into ethyl acetate and add 120 g of silica gel to the ethyl acetate solution. Place the mixture atop a column of 1500 g of silica gel and elute with 20% ethyl acetate/benzene to obtain the pure diol. (m.p. 131—133°C).

Step D: 9-Nitro-6H-dibenz[b,e][1,4]oxathiepin

Stir 4.5 g of the diol of Step C and 17.1 g (5 molar equivalents) of dicyclohexylcarbodiimide together at 110—115°C for 4—5 hours. Cool the mixture, dissolved in 250 ml of tetrahydrofuran and filter. Add silica gel

to the filtrate and strip to dryness. Place the residue atop a column of 310 g of silica gel and elute with 50:50 benzene/hexane. Strip to dryness to obtain the title product. (m.p. 112—113°C).

Step E: 9-Amino-6H-dibenz[b,e][1,4]oxathiepin

Dissolve 7.92 g of the nitro compound of Step D in 150 ml of tetrahydrofuran and add 50 ml of concentrated hydrochloric acid. Place the mixture in a cold water bath and add 22.7 g (3 molar equivalents + 10%) of stannous chloride dihydrate in portions. Stir at room temperature for 5½ hours. Dilute the reaction mixture with water, basify with 40% aqueous sodium hydroxide and extract with ethyl acetate. Wash the organics with water, dry and strip to dryness. (Yield: 7.22 g crude amine).

Step F: 9-Cyano-6H-dibenz[b,e][1,4]oxathiepin

Suspend 1.55 g (6.77 mmoles) of the amine of Step E in 36 ml of 1N hydrochloric acid and cool the mixture in an ice bath. Add slowly a solution of 502 mg (7.28 mmoles) of sodium nitrite in 10 ml of water, keeping the temperature at 0—5°C. Stir the mixture in the cold for 15 minutes. Neutralize to pH 7 with aqueous sodium carbonate solution. Add the mixture slowly to a cooled mixture of 1.37 g (15.3 mmoles) of cuprous cyanide and 2.0 g (30.8 mmoles) of potassium cyanide in 50 ml of water at 0—5°C. Recover the precipitate by filtration and wash well with water. Dissolve the precipitate in tetrahydrofuran, add silica gel and evaporate the tetrahydrofuran. Place the residue atop a silica gel column and elute with 50:50 benzene/hexane. Remove the solvent to obtain the title product. (m.p. 136—137°C).

Step G: 9-Cyano-6H-dibenz[b,e][1,4]oxathiepin-11,11-dioxide

Dissolve 850 mg (3.56 mmole) of the 9-cyano-6H-dibenz[b,e][1,4]oxathiepin of Step F in 50 ml of methylene chloride. Add 2.3 g (11.3 mmole) of 85% m-chloroperbenzoic acid and stir at room temperature for 2 hours. Add excess calcium hydroxide and continue stirring for a few minutes. Filter the reaction mixture through celite and strip the filtrate to dryness. Chromatograph the residue on silica gel eluting with 25% ethyl acetate in benzene to obtain the title product. (m.p. 177—179°C).

Step H: 9-(1H-Tetrazol-5-yl)-6H-dibenz[b,e][1,4]oxathiepin-11,11-dioxide

Heat together 435 mg (1.61 mmole) of the nitrile of Example 2, 156 mg (2.4 mmole) of sodium azide and 161 mg (3 mmole) of ammonium chloride in 10 ml of dimethylformamide at 135°C for 6 hours. Cool the reaction mixture to room temperature, dilute with water and excess aqueous sodium carbonate solution and extract with ethyl acetate. Acidify and extract the separated solid with ethyl acetate or triturate in ether. Filter and dissolve the product in 40 ml of hot methanol. Filter and concentrate the filtrate to 15 ml. Cool and scratch to induce crystallization. Dry under vacuum at 130°C to obtain the title product. (m.p. dec. 225°C).

Example 2

9-(1H-Tetrazol-5-yl)-6H-dibenz[b,e][1,4]oxathiepin

Heat a mixture of 800 mg (3.35 mmole) of 9-cyano-6H-dibenz[b,e][1,4]oxathiepin (see Example 1 Step F), 293 mg (4.5 mmole) of sodium azide and 265 mg (4.95 mmole) of ammonium chloride in 25 ml of dimethylformamide at 130—135°C for 6 hours. Dilute the mixture with water and excess sodium carbonate. Extract with ethyl acetate. Acidify the aqueous phase and separate the precipitate by filtration to obtain the title product. (m.p. melts with dec. 195—200°C).

Example 3

9-(1H-Tetrazol-5-yl)-6H-dibenz[b,e][1,4]oxathiepin-11-oxide

Dissolve with warming 500 mg (1.77 mmole) of the tetrazolyl compound of Example 2 in 50 ml of glacial acetic acid. Place the reaction mixture in an oil bath at 45°C and, after equilibration, add 2 ml of 30% hydrogen peroxide. Stir at 45°C for several hours until the sulfoxide crystallizes out. (m.p. dec. 292—295°C).

Example 4

2-(1H-Tetrazol-5-yl)-6H-dibenz[b,e][1,4]oxathiepin

Step A: 2-(o-Methoxyphenylthio)benzoic acid

Stir under reflux for 3 hours a mixture of 70 g (0.5 mole) of o-methoxythiophenol, 120.5 g (0.486 mole) of o-iodobenzoic acid, 81.7 g (1.46 mole) of potassium hydroxide, 85 g (1.34 mole) of copper powder and 800 ml of water. Filter the reaction mixture hot and again filter the filtrate through celite. Acidify the filtrate with concentrated hydrochloric acid. Separate the precipitate, wash well with water and dry *in vacuo* at 70°C to obtain the title product. (m.p. 198—200°C).

Step B: Methyl 2-(o-methoxyphenylthio)benzoate

Dissolve 115 g of the acid of Step A in 3.5 litres of methanol and add slowly 25 ml of sulfuric acid. Stir under reflux for 72 hours. Cool the reaction mixture to room temperature and add 100 g of sodium bicarbonate in portions. Stir for an additional hour and strip to dryness. Dissolve the residue in methylene chloride and wash the solution three times with water. Dry the solution and strip to an oil which solidifies. (m.p. 82—84°C).

Step C: 3-(o-Carbomethoxyphenylthio)-4-methoxybenzaldehyde

Dissolve 117 g (0.427 mole) of the ester of Step B in 1500 ml of 1,2-dichloroethane and cool with stirring in an ice-bath. Add 200 ml (345 g, 1.82 mole) of titanium tetrachloride at a rapid dropwise rate. Add also fairly rapidly 154 g (1.34 mole) of dichloromethyl methyl ether. Stir the mixture under a nitrogen atmosphere overnight then pour into ice. After shaking, separate the organic phase and extract the aqueous phase twice with methylene chloride. Wash the combined organic phases twice with water, dry and strip to an oil which crystallizes. (m.p. 99—104°C).

Step D: 3-(o-Carboxyphenylthio)-4-hydroxybenzaldehyde

Heat 126 g of the aldehyde of Step C in a mixture of 1500 ml of glacial acetic acid and 1500 ml of 48% hydrogen bromide in an oil bath at 150°C with mechanical stirring until no trace of nondemethylated product remains (4—5 days). Cool the reaction mixture and pour into 7 liters of water. Separate the precipitate, wash well with water and dry *in vacuo* at 70°C to constant weight. (Yield: 108.2 g).

Step E: 3-(o-Carboxyphenylthio)-4-hydroxybenzonitrile

Reflux 91.3 g of the aldehyde of Step D, 27.4 g of hydroxylamine hydrochloride and 41.9 g of sodium formate in 900 ml of formic acid (98—100%) for 1¼ hours. Cool the mixture and pour into 2½ liters of cold water. Separate the precipitate, wash with water and dry *in vacuo* at 75°C. (Yield: 82 g).

Step F: 2-Cyano-6H-6-oxo-dibenzo[b,e][1,4]oxathiepin

Stir together at room temperature overnight 8.4 g of the nitrile of Step E and 19.16 g (3 molar equivalents) of dicyclohexylcarbodiimide in 400 ml of ethyl acetate. Filter the reaction mixture to remove the urea. Strip the filtrate to dryness. Triturate the residue in a small volume of ethyl acetate and filter (Yield 5.5 g purple solid).

Strip the filtrate to dryness and chromatograph on a column of silica gel, eluting with benzene. (Yield 2.1 g white solid), (yield total: 7.6 g).

Step G: 3-(o-Hydroxymethylphenylthio)-4-hydroxybenzonitrile

Dissolve 31.25 g (0.123 mole) of the nitrile of Step F in 750 ml of tetrahydrofuran and add 10.4 g (0.274 moles) of sodium borohydride. Stir the solution at room temperature for 1½ hours. Add water in small portions until foaming ceases. Remove the tetrahydrofuran by evaporation. Shake the residue with ethyl acetate, water and dilute hydrochloric acid. Separate the organic phase and extract the aqueous phase three times with ethyl acetate. Wash the combined organic phases with two small volumes of water, dry and strip to a thick oil which solidifies. (Yield: 39 g).

Step H: 2-Cyano-6H-dibenz[b,e][1,4]oxathiepin

Stir the crude nitrile of Step F (assumed 100% yield, 0.123 mole) and 38 g (50% excess) of dicyclohexylcarbodiimide at 105—110°C for 1½ hours. Cool the reaction mixture and extract with methylene chloride. Filter to remove the dicyclohexylurea and strip the filtrate to dryness. Triturate in a small volume of ethyl acetate, filter and strip to dryness. Extract four times with hot benzene and strip the combined extracts to dryness. Chromatograph on a silica gel column, eluting with benzene to obtain the title product. (m.p. 145—147°C).

Step I: 2-(1H-Tetrazol-5-yl)-6H-dibenz[b,e][1,4]oxathiepin

Reflux 3 g (12.55 mmole) of the nitrile of Step F, 1 g (15.4 mmole) of sodium azide and 1 g (18.7 mmole) of ammonium chloride in 40 ml of dimethylformamide overnight. Cool to room temperature. Add aqueous sodium bicarbonate and ethyl acetate and stir the mixture for 30 minutes. Separate the aqueous phase and extract with ethyl acetate. Acidify the aqueous phase and separate the title product by filtration. (m.p. 230°C dec.).

### Example 5

### 2-(1H-Tetrazol-5-yl)-6H-dibenz[b,e][1,4]oxathiepin-11-oxide

Suspend 2 g of the tetrazolyl compound of Example 4 in 120 ml of acetic acid. Add 15 ml of 30% hydrogen peroxide. Place the reaction mixture in an oil bath and heat slowly to 60°C. Stir at 60°C for 2 hours. Cool to room temperature, filter wash the precipitate with acetic acid and dry to obtain the title product. (m.p. 282°C dec.).

### Example 6

### 2-(1H-Tetrazol-5-yl)-6H-dibenz[b,e][1,4]oxathiepin-11,11-dioxide

Suspend 600 mg of the tetrazole compound of Example 4 in 40 ml of glacial acetic acid and add 5 ml of 30% hydrogen peroxide. Heat to 70—75°C for 2½ hours until a clear solution is obtained. Cool the reaction mixture to room temperature. Separate the precipitated title product, wash with acetic acid and air dry. (m.p. 255°C dec.).

Example 7

Methyl 6H-dibenz[b,e][1,4]-oxathiepin-9-carboxylate

Step A: 9-Chlorocarbonyl-6H-dibenz[b,e][1,4]oxathiepin

Dissolve 5.16 g of 6H-dibenz[B,e][1,4]oxathiepin-9-carboxylic acid in 100 ml of chloroform and 50 ml of thionyl chloride and add to the mixture 1.0 ml of dimethylformamide. Allow the mixture to stand at room temperature of 72 hours. Evaporate the mixture to dryness to obtain the desired acid chloride.

Step B: Methyl 6H-dibenz[b,e][1,4]oxathiepin-9-carboxylate

Dissolve 2.0 g of the acid chloride of Step A in 20 ml of tetrahydrofuran containing 1.0 ml of methanol and 4 ml of pyridine. Allow the mixture to stand at room temperature for 24 hours then evaporate to dryness. Dissolve the residue in 1:4 ether/hexane and filter through silica gel. Evaporate the filtrate to dryness to obtain the title product.

Carrying out the process of Example 7, but substituting another $C_{1-4}$ alkanol, for example, ethanol, 2-propanol, butanol or 2-butanol, for the methanol of step B, the corresponding $C_{1-4}$ alkyl ester of 6H-dibenz[b,e][1,4]-oxathiepin-9-carboxylic acid is obtained.

Example 8

6H-Dibenzo[b,e][1,4]oxathiepin-2-carboxamide

Step A: 2-Chlorocarbonyl-6H-dibenz[b,e][1,4]oxathiepin

Heat a solution of 5 g of 6H-dibenz[b,e][1,4]oxathiepin-2-carboxylic acid and 40 ml of thionyl chloride under reflux for 20 minutes. Evaporate the reaction mixture under vacuum to dryness. Repeat the evaporation with two 30 ml portions of carbon tetrachloride. Crystallize the residue from diisopropyl ether to obtain the title product.

Step B: 6H-Dibenzo[b,e][1,4]oxathiepin-2-carboxamide

Dissolve the acid chloride from Step A in 20 ml of dry tetrahydrofuran and add this solution dropwise with stirring to a cooled (ice-bath) saturated solution of ammonia in 60 ml of tetrahydrofuran. Pass ammonia through the reaction mixture continuously for 15 minutes. Stir at room temperature for an additional 15 minutes and evaporate the reaction mixture to dryness. Add a mixture of 12 ml of ethanol and 60 ml of water to the residue and stir at room temperature for an additional 30 minutes. Separate the solid by filtration and wash with water, then with ethanol and then with ether. Dry *in vacuo* to obtain the title product.

In a similar manner, substituting 6H-dibenz[b,e][1,4]oxathiepin-9-carboxylic acid for the 6H-dibenz-[b,e][1,4]oxathiepin-2-carboxylic acid in Sep A, there is obtained 6H-dibenz[b,e][1,4]oxathiepin-9-carboxamide.

Example 9

6H-Dibenzo[b,e][1,4]oxathiepin-2-N-methylcarboxamide

Add 6.0 g of 2-chlorocarbonyl-6H-dibenz[b,e][1,4]oxathiepin to 4 g of methylamine in 100 ml of methylene chloride at 0—5°C. Add 13 ml of triethylamine dropwise over 10 minutes then stir the reaction mixture at room temperature overnight. Extract the reaction mixture with water, dry the organic layer and evaporate to dryness. Chromatograph over silica gel eluting with 200:20 toluene/dioxane. Evaporate eluate to dryness and recrystallize the residue from methanol to obtain the title product.

In a similar manner, substituting another $C_{1-4}$ N-alkylamine, for example, ethylamine, propylamine, isopropylamine or butylamine, or an N,N-di-($C_{1-4}$ alkyl)amine, for example, dimethylamine, diethylamine, dipropylamine or dibutylamine, for the methylamine used above, there is obtained the corresponding 6H-dibenz[b,e][1,4]oxathiepin-2-N-alkylcarboxamide or 2-N,N-di-alkylcarboxamide. Corresponding 6H-dibenz-[b,e][1,4]oxathiepin-9-N-alkylcarboxamides and 9-N,N-di-aklylcarboxamides are prepared by substituting 9-chlorocarbonyl-6H-dibenz[b,e][1,4]oxathiepin for the 2-chlorocarbonyl-6H-dibenz[b,e][1,4]oxathiepin used above.

Also in a similar manner, substituting a carboxy($C_{1-4}$ alkyl)amine, for example, glycine, valine, leucine or isoleucine or an N-($C_{1-4}$ alkyl) derivative thereof, for example, N-methylglycine, N-propyleucine or N-butylisoleucine, there is obtained the corresponding 6H-dibenz[b,e][1,4]oxathiepin-9-(or 2-)carboxyalkyl-carboxamide or N-alkyl derivative.

Example 10

6H-Dibenz[b,e][1,4]oxathiepin-2-N-methanesulphonyl-carboxamide

Heat 5.0 g of 6H-dibenz[b,e][1,4]oxathiepin-2-carboxylic acid in 50 ml of thionyl chloride for 15 minutes at reflux and then distil off the excess of thionyl chloride. Evaporate the residue twice with small volumes of benzene. Add the resulting acid chloride to 4.0 g of methanesulphonamide in 100 ml of methylene chloride at 0—5°C. Add dropwise over 10 minutes 15 ml of triethylamine. Stir the mixture at room temperature overnight. Extract the reaction mixture with 100 ml of 0.5 N sodium hydroxide, wash the alkaline extract with ether and acidify with 6N hydrochloric acid. Separate the solids by filtration and dry *in vacuo* over potassium hydroxide. Chromatograph over silica gel eluting with 200:20:3 toluene/dioxane/acetic acid. Evaporate the eluate to dryness and recrystallize the residue from methanol to obtain the product.

In a similar manner, substituting another $C_{1-4}$ alkylsulphonamide, for example ethanesulphonamide, propanesulphonamide or butanesulphonamide, for the methanesulphonamide used above, there is obtained the corresponding 6H-dibenzo[b,e][1,4]oxathiepin-2-N-alkylsulfonylcarboxamide. Corresponding 6H-dibenz[b,e][1,4]oxathiepin-9-N-alkylsulphonylcarboxamides are prepared by substituting 6H-dibenz-[b,e][1,4]-oxathiepin-9- carboxylic acid for the 6H-dibenz[b,e][1,4]-oxathiepin-2-carboxylic acid used above.

Example 11

6H-Dibenz[b,e][1,4]oxathiepin-2-(3-methyl-2-thiazolidinylidene)carboxamide

Reflux 1.0 g of 6H-dibenz[b,e][1,4]oxathiepin-2-carboxylic acid in 15 ml of thionyl chloride for 30 minutes. Strip the reaction mixture to dryness and dissolve the residue in 25 ml of methylene chloride. Add a solution of 1.0 g of 2-imino-3-methylthiazolidine in 10 ml of methylene chloride. Stir at room temperature for 30 minutes and add water. Continue stirring for 10 minutes. Separate the organic phase and wash with water and dry overnight over sodium sulfate. Strip to dryness. Stir and triturate the residue in ether, then in methanol. Chromatograph the resulting solid over silica gel, eluting with 20% ethylacetate in benzene. Strip to dryness to obtain the title product.

Example 12

2-(3-Hydroxy-1,2,5-thiadiazol-4-yl)-6H-dibenz[b,e][1,4]oxathiepin

Step A: 6H-Dibenz[b,e][1,4]oxathiepin-1-carboxaldehyde

Heat a mixture of 5.0 g of 2-cyano-6H-dibenz[b,e][1,4]oxathiepin and 4.0 g of Raney nickel alloy in 60 ml of 75% (v/v) aqueous formic acid at reflux for 1.5 hours. Cool to room temperature and filter. Concentrate to small volume and extract with methylene chloride. Wash the extract with water and with 1N sodium bicarbonate until neutral. Dry the neutral extract over sodium sulfate and concentrate to dryness to obtain the title product.

Step B: 6H-Dibenz[b,e][1,4]oxathiepin-2-(2-aminoacetonitrile)

Stir at room temperature for 12 hours a mixture of 5.85 g of ammonium chloride, 5.4 g of sodium cyanide 75 ml of ammonium hydroxide, 100 ml of ethanol saturated with ammonia and 12 g of the carboxaldehyde of Step A. Pour the reaction mixture into 300 ml of water and extract with ether. Dry the extract over sodium sulfate and concentrate to dryness to obtain the title product.

Step C: 6H-Dibenz[b,e][1,4]oxathiepin-2-(2-aminoacetamide)

Stir at room temperature 5.0 g of the aminoacetonitrile of Step B in 30 ml of concentrated hydrochloric acid for 30 minutes. Slowly pour the reaction mixture into cold ammonium hydroxide. Extract the mixture with ether and dry over sodium sulfate. Evaporate the extract to dryness to obtain the title product.

Step D: 2-(3-Hydroxy-1,2,5-thiadiazol-4-yl)-6H-dibenz[b,e][1,4]oxathiepin

Stir overnight at room temperature a mixture of 1.365 g of the aminoacetamide of Step C, 1.989 g of sulfur monochloride and 5 ml of dimethylformamide. Filter the reaction mixture and then partition between ice-water (75 ml) and ethyl acetate (75 ml). Filter, separate the organic layer, wash with saturated aqueous sodium chloride solution and dry over magnesium sulfate. Evaporate to dryness and dissolve the residue in 200 ml of boiling ethanol, treat with charcoal and filter. Concentrate to 25 ml, and separate the solids by filtration to obtain the title product.

By substituting 9-cyano-6H-dibenz[b,e][1,4]oxathiepin for the 2-cyano-6H-dibenz[b,e][1,4]oxathiepin used in Step A above, there is obtained the corresponding 9-(3-hydroxy-1,2,5-thiadiazol-4-yl)-6H-dibenz-[b,e][1,4]oxathiepin.

Example 13

2-(4-Hydroxy-$\Delta^3$-pyrrolin-3-yl-2,5-dione)-6H-dibenz[b,e][1,4]oxathiepin

Step A: 2-Hydroxymethyl-6H-dibenz[b,e][1,4]oxathiepin

Dissolve 5.1 g of 6H-dibenz[b,e][1,4]oxathiepin-2-carboxylic acid in 100 ml of tetrahydrofuran and add 35 ml of 1M borane in tetrahydrofuran at room temperature under a nitrogen atmosphere. Stir the mixture at room temperature for 3 hours. Slowly dilute the reaction mixture with water and then with ethyl acetate. Wash with aqueous sodium chloride, dry and evaporate to an oil. Yield 4.93 g (crude).

Step B: 2-Bromomethyl-6H-dibenz[b,e][1,4]oxathiepin

Dissolve 4.43 g of the crude alcohol of Step A in 100 ml of benzene and add 1 ml (10.5 mmole) of phosphorous tribromide. Stir at room temperature for 1 hour, add water and then dilute with toluene. Wash three times with water, dry and strip to a solid residue. (Yield, 6.9 g, holding toluene)

Step C: 2-Cyanomethyl-6H-dibenz[b,e][1,4]oxathiepin

Dissolve 6.4 g of the bromide of step in 75 ml of dimethylformamide and add 2.95 g of sodium cyanide. Stir the mixture at room temperature for 1.5 hours. Dilute with 600 ml of water and extract three times with ether. Wash the combined organics with water, dry and strip to a solid residue. Triturate in hexane and recover the solid by filtration. (m.p. 105—107°C.)

Step D: 6H-Dibenzo[b,e][1,4]oxathiepin-2-acetic acid

Reflux 2.0 g of the nitrile of Step C in a mixture of 30 ml of 20% aqueous sodium hydroxide and 30 ml of ethanol for four hours. Strip away the alcohol, wash with ethyl acetate and acidify the aqueous phase with hydrochloric acid. Separate the precipitate by filtration. Wash with water and dry. (m.p. 144—146°C.)

Step E: 6H-Dibenzo[b,e][1,4]oxathiepin-2-acetamide

Reflux for 20 minutes a mixture of 5.0 g of the acid of Step D and 40 ml of thionyl chloride. Evaporate to dryness under vacuum. Evaporate twice with 30 ml portions of carbon tetrachloride. Dissolve the residue in 20 ml of tetrahydrofuran and add the solution dropwise to a cooled and stirred saturated solution (ice-bath) of ammonia in 60 ml of tetrahydrofuran. Pass ammonia through the solution simultaneously. Continue stirring at room temperature for an additional 15 minutes. Evaporate the mixture to dryness. Add a mixture of 12 ml of ethanol and 60 ml of water and stir the suspension for 30 minutes. Separate the solids and wash with water, then with ethanol and finally with ether to obtain the title product.

Step F: 2-(4-Hydroxy-$\Delta^3$-pyrrolin-3-yl-2,5-dione)-6H-dibenz[b,e][1,4]oxathiepin

Stir at room temperature a mixture of 5.118 g of the amide of Step E, 2.939 g of diethyl oxalate, 4.723 g of potassium *t*-butoxide and 40 ml of dimethylformamide for 6 hours. Pour the reaction mixture into 300 ml of ice-water and extract with 300 ml of ethyl acetate. Acidify with 6N hydrochloric acid and separate the ethyl acetate layer. Wash with saturated sodium chloride solution and dry. Evaporate to dryness and dissolve the residue in warm dioxane. Treat with a slight excess of ammonia and separate the solid by filtration. Wash with dioxane and dry. Suspend the product in water, acidify with 6N hydrochloric acid and extract with ethyl acetate. Wash the extract with saturated sodium chloride solution, dry over magnesium sulfate and evaporate to obtain the title product.

Example 14

β-Hydroxyethyl 6H-dibenz[b,e][1,4]oxathiepin-2-carboxylate

To a stirred solution of 1.0 g of 2-chlorocarbonyl-6H-dibenz[b,e][1,4]oxathiepin in 50 ml of methylene chloride, add 3 g of ethylene glycol and stir the mixture for 18 hours at room temperature. Distill off the solvent and excess ethylene glycol under high vacuum (0.1 mm). Chromatograph the residue on a silica gel column (100 g), eluting with 10% ethyl acetate in benzene to obtain the title product.

In a similar manner, substituting another loweralkyldiol such as, for example, trimethylene glycol and 1,4-butanediol and the like for the ethylene glycol, there is obtained the corresponding hydroxyloweralkylester. The corresponding hydroxyloweralkyl 9-carboxylate esters are prepared by substituting 9-chlorocarbonyl 6H-dibenz[b,e][1,4]oxathiepin for the 2-chlorocarbonyl 6H-dibenz[b,e][1,4]oxathiepin employed above.

Example 15

β-Dimethylaminoethyl 6H-dibenz[b,e][1,4]oxathiepin-9-carboxylate

Dissolve 1.0 g of 9-chlorocarbonyl-6H-dibenz[b,e][1,4]oxathiepin as prepared in Example 7, Step A, in 10 ml of anhydrous tetrahydrofuran with stirring and add 2 ml of N,N-dimethylethanolamine. Stir at room temperature for 18 hours and strip the mixture to dryness. Partition the residue between ether and dilute hydrochloric acid and separate the aqueous layer. Basify the aqueous layer with aqueous ammonia and extract with ethyl acetate. Evaporate the organic phase and chromatograph the residue over silica-gel eluting with 90% chloroform in methanol to obtain the title product.

In a similar manner, substituting another N,N-di($C_{1-4}$ alkyl)amino($C_{1-4}$ alkanol), for example diethyl-ethanolamine, 3-N,N-dimethylamino-propan-1-ol or 4-N,N-diethylaminobutan-1-ol, for the N,N-dimethylethanolamine there is obtained the corresponding N,N-dialkylaminoalkyl ester. The corresponding N,N-dialkyl 2-carboxylate esters are prepared by substituting 2-chlorocarbonyl-6H-dibenz[b,e][1,4]oxathiepin for the 9-chlorocarbonyl-6H-dibenz[b,e][1,4]oxathiepin used above.

Example 16

6H-Dibenz[b,e][1,4]oxathiepin-2-N-carboxy($C_{1-4}$ alkyl)carboxamides

Reflux 1.0 g of 2-chlorocarbonyl-6H-dibenz[b,e][1,4]oxathiepin in 20 ml of ethyl acetate containing 2.0 g of glycine for 5 hours. Evaporate the mixture to dryness. Add 30 ml of water to the solid residue and stir at room temperature for one hour. Separate the solid by filtration and recrystallize from ethanol to obtain the title product.

In a similar manner, substituting another amino acid, for example, alanine or valine for the glycine, there is obtained the corresponding 2-carboxy($C_{1-4}$alkyl)carboxamide.

The corresponding 9-carboxyalkylcarboxamides are prepared by substituting 9-chlorocarbonyl-6H-dibenz[b,e][1,4]oxathiepin for the 2-chlorocarbonyl-6H-dibenz[b,e][1,4]oxathiepin used above.

Example 17

β-Carboxyethyl 6H-dibenz[b,e][1,4]oxathiepin-2-carboxylate

Dissolve 1.0 g of 2-chlorocarbonyl-6H-dibenz[b,e][1,4]oxathiepin in 20 ml of tetrahydrofuran and add

1.0 g of the sodium salt of β-hydroxypropionic acid. Stir the mixture at room temperature for 18 hours. Filter and evaporate the filtrate to dryness. Recrystallize the solid residue from ethanol to obtain the title product.

In a similar manner, substituting another $C_{1-4}$ hydroxyalkanoic acid salt, for example, an alkali metal salt of hydroxyacetic acid or 3-hydroxy-butyric acid, for the β-hydroxypropionic acid sodium salt, there is obtained the corresponding carboxyalkyl-2-carboxylate ester. The corresponding carboxyalkyl-9-carboxylate esters are prepared by substituting 9-chlorocarbonyl-6H-dibenz[b,e][1,4]oxathiepin for the 2-chlorocarboxyl-6H-dibenz[b,e][1,4]oxathiepin used above.

### Example 18
### 2-(1H-Tetrazol-5-yl-methyl)-6H-dibenz[b,e][1,4]oxathiepin

Add to 25 ml of tetrahydrofuran cooled in an ice-bath 1.59 g (11.9 mmole) of aluminium chloride, 1.33 g (5.25 mmole) of 2-cyanomethyl-6H-dibenz[b,e][1,4]oxathiepin and 1.55 g (23.8 mmole) of sodium azide. Reflux the mixture for 19 hours, cool, dilute with water and acidify. Extract the mixture into ethyl acetate and evaporate. Triturate the residue in ether and separate the title product by filtration. (m.p. 193—195°C).

### Example 19
### 6H-Dibenz[b,e][1,4]oxathiepin-2-acetic acid-11,11-diode

Heat 600 mg of 6H-dibenz[b,e][1,4]oxathiepin-2-acetic acid to 80—85°C in a mixture of 30 ml of glacial acetic acid and 5 ml of 30% hydrogen peroxide for 3 hours. Dilute with water to final volume of about 250 ml. Separate the title product by filtration. (m.p. 188—190°C).

### Example 20
### N-(Carboxymethyl)-6H-dibenz[b,e][1,4]oxathiepin-2-carboxamide-11,11-dioxide

Step A: N-(Carbomethoxymethyl)-6H-dibenz[b,e][1,4]oxathiepin-2-carboxamide-11,11-dioxide

Suspend 1.161 g of 6H-dibenz[b,e][1,4]oxathiepin-2-carboxylic acid-11,11-dioxide in 70 ml THF and add 2.52 ml of triethylamine followed by 0.43 ml of ethylchloroformate. Stir the mixture at room temperature for 5 minutes. Add in portions 1.134 g glycine methyl ester HCl. Stir at room temperature for twenty-five hours. Filter and wash the collected solids with THF. Evaporate the combined THF and filtrate to dryness. Dissolve the residue in methylene chloride, add 10 g silica gel and evaporate the methylene chloride. Place the residue atop a silica gel column and elute with 50:50 ethyl acetate/toluene. Remove the solvent to obtain the title product. (m.p. 169—172°C).

Step B: N-(Carboxymethyl)-6H-dibenz[b,e][1,4]oxathiepin-2-carboxamide-11,11-dioxide

Suspend 542 mg of the methyl ester from Step A and 84 mg of lithium hydroxide hydrate in a mixture of 10 ml water and 10 ml THF. Stir at room temperature for 1.5 hours. Dilute the mixture with 20 ml of water acidified with 2*N* HCl and extract the mixture with ethyl acetate. Wash the ethyl acetate extracts with saturated sodium chloride solution and dry. Evaporate the solution to dryness. Triturate the resulting solid with ether to obtain the title product. (m.p. 235°C).

### Example 21
### 2-[1-(Pivalyloxy)ethoxycarbonyl]-6H-dibenz[b,e][1,4]oxathiepin-11,11-dioxide

To a mixture of 204 mg 6H-dibenz[b,e][1,4]oxathiepin-2-carboxylic acid-11,11-dioxide (0.7 mmole) and 1.5 g of anhydrous potassium carbonate in 10 ml of dimethyl formamide, there was added a solution of 390 mg 1-chloroethyl pivalate (2.3 mmole) in 10 ml of dimethyl formamide. The reaction mixture was stirred at ambient temperature for 24 hours, then it was partitioned between water and ethyl acetate. The crude product obtained from evaporation of the organic phase was chromatographed on a column of silica gel, eluting with dichloromethane, to isolate the ester which was stirred in a small amount of ether for 4 hours then filtered. The yield was 141 mg, m.p.: 136—137°C.

Although the invention has been described above in terms of the use of the novel oxathiepins disclosed herein in the treatment and control of human and warm-blooded animal disease conditions characterized by excessive undesirable contractile activity of prostaglandins and prostaglandin biosynthetic intermediates, and particularly of asthma, it will be recognized that contractile prostaglandins are involved not only in chronic obstructive lung disease (e.g. asthma), but also in other allergic conditions as well as in inflammation, diarrhoea, hypertension, angina, platelet aggregation, cerebral spasm, premature abortion and dismenorrhoea. Also oxathiepins of this invention are potent $TXA_2$ biosynthesis inhibitors, inhibiting platelet aggregation, and can be useful in diseases such as atherosclerosis, and myocaridial infarction.

Certain of the compounds of the present invention can be separated into optically active (+) and (−) isomers. In such cases, the present invention includes both the racemic as well as the resolved forms of the compound.

**Claims**

1. A 6H-dibenz[b,e][1,4]oxathiepin having the structural formula:

I 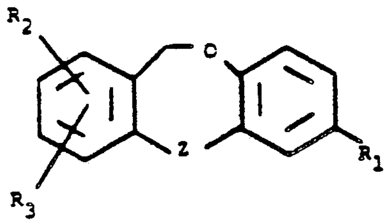 or 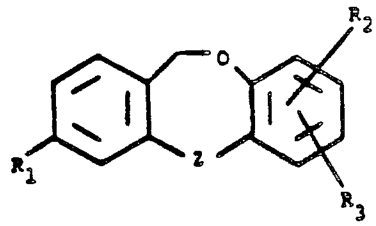 Ia

in which

Z is thia, sulfinyl, or sulfonyl;

each of $R_2$ and $R_3$, which are the same or different, is hydrogen, halogen, nitro, $C_{1-4}$ alkyl, amino, $C_{1-4}$ N-alkylamino, N,N-di($C_{1-4}$ alkyl)amino, $C_{1-4}$ alkanoyl, hydroxy, $C_{1-4}$ alkoxy, $C_{1-4}$ alkylthio, trifluoromethylthio, $C_{1-4}$ alkylsulfinyl, $C_{1-4}$ alkylsulfonyl, or trifluoromethyl;

$R_1$ is 5-tetrazolyl, 5-tetrazolylmethyl, 3-hydroxy-1,2,5-thiadiazol-4-yl, 4-hydroxy-$\Delta^3$-pyrrolin-3-yl-2,5-dione or

$$(CH_2)_n-\overset{O}{\overset{\|}{C}}-R_4$$

where n is 0 or an integer of from 1 to 4 and $R_4$ is N,N-di($C_{1-4}$ alkyl)amino-($C_{1-4}$ alkoxy), $C_{1-4}$ hydroxy alkoxy, carboxy-($C_{1-4}$ alkoxy), amino, $C_{1-4}$ N-alkylamino, N,N-di($C_{1-4}$ alkyl)amino, $C_{1-4}$ alkylsulfonylamino, carboxy($C_{1-4}$ alkyl)amino, carboxamido-($C_{1-4}$ alkyl)amino, 2-imino-3-methylthiazolidine, ($C_{1-4}$ acyloxy)-($C_{1-4}$ alkoxy) or (5-methyl-2-oxo-1,3-dioxolan-4-yl)methoxy);
and compounds that are pharmaceutically acceptable salts thereof.

2. A compound of Claim 1 having the name:
9-(1H-Tetrazol-5-yl)-6H-dibenz[b,e][1,4]oxathiepin-11,11-dioxide;
9-(1H-Tetrazol-5-yl)-6H-dibenz[b,e][1,4]oxathiepin-11-oxide;
2-(1H-Tetrazol-5-yl)-6H-dibenz[b,e][1,4]oxathiepin;
2-(1H-Tetrazol-5-yl)-6H-dibenz[b,e][1,4]oxathiepin-11-oxide;
2-(1H-Tetrazol-5-yl)-6H-dibenz[b,e][1,4]oxathiepin-11,11-dioxide;
Methyl 6H-dibenz[b,e][1,4]oxathiepin-9-carboxylate;
6H-Dibenzo[b,e][1,4]oxathiepin-2-carboxamide;
6H-Dibenzo[b,e][1,4]oxathiepin-2-N-methylcarboxamide;
6H-Dibenz[b,e][1,4]oxathiepin-2-N-methanesulfonyl-carboxamide;
6H-Dibenz[b,e][1,4]oxathiepin-2-(3-methyl-2-thiazolidinylidene)carboxamide;
2-(3-Hydroxy-1,2,5-thiadiazol-4-yl)-6H-dibenz[b,e][1,4]oxathiepin;
2-(4-Hydroxy-$\Delta^3$-pyrrolin-3-yl-2,5-dione)-6H-dibenz[b,e][1,4]oxathiepin;
β-Hydroxyethyl 6H-dibenz[b,e][1,4]oxathiepin-2-carboxylate;
β-Dimethylaminoethyl 6H-dibenz[b,e][1,4]oxathiepin-9-carboxylate;
β-Carboxyethyl 6H-dibenz[b,e][1,4]oxathiepin-2-carboxylate;
2-(1H-Tetrazol-5-ylmethyl)-6H-dibenz[b,e][1,4]oxathiepin;
6H-Dibenz[b,e][1,4]oxathiepin-2-acetic acid-11,11-dioxide;
N-(Carboxymethyl)-6H-dibenz[b,e][1,4]oxathiepin-2-carboxamide-11,11-dioxide;
2-[1-(Pivaloyloxy)ethoxycarbonyl]-6H-dibenz[b,e][1,4]oxathiepin-11,11-dioxide;
Methyl 6H-dibenz[b,e][1,4]oxathiepin-2-carboxylate-11,11-dioxide;
Methyl 9-nitro-6H-dibenz[b,e][1,4]oxathiepin-2-carboxylate;
Methyl 9-amino-6H-dibenz[b,e][1,4]oxathiepin-2-carboxylate;
Methyl 8-amino-6H-dibenz[b,e][1,4]oxathiepin-2-carboxylate;
Methyl 8-nitro-6H-dibenz[b,e][1,4]oxathiepin-2-carboxylate;
6H-Dibenz[b,e][1,4]oxathiepin-2-acetic acid;
6H-Dibenz[b,e][1,4]oxathiepin-2-acetamide; or
9-(1H-Tetrazol-5-yl)-6H-dibenz[b,e][1,4]oxathiepin.

3. A compound as claimed in Claim 1 in which Z is thio.

4. A compound as claimed in Claim 3 in which $R_1$ is 5-tetrazolyl or

$$-(CH_2)_n-\overset{O}{\overset{\|}{C}}-R_4$$

where $R^4$ is as defined in Claim 1.

5. A compound as claimed in Claim 4 in which $R_2$ is hydrogen, amino, nitro or fluoro and $R_3$ is hydrogen; and $R_1$ is 5-tetrazolyl or

$$\overset{\displaystyle O}{\overset{\|}{-C-R_4}}$$

where $R^4$ is as defined in Claim 1.

6. A compound as claimed in Claim 5 in which $R_2$ is hydrogen and $R_1$ is 5-tetrazolyl.

7. A compound as claimed in Claim 5 in which $R_1$ is

$$\overset{\displaystyle O}{\overset{\|}{-C-R_4}}$$

where $R_4$ is as defined in Claim 1.

8. A compound as claimed in Claim 1 in which Z is sulfinyl and $R_1$, $R_2$ and $R_3$ are as defined in Claim 5.

9. A compound as claimed in Claim 8 in which $R_2$ is hydrogen and $R_1$ is 5-tetrazolyl.

10. A compound as claimed in Claim 8 in which $R_1$ is

$$\overset{\displaystyle O}{\overset{\|}{-C-R_4}}$$

where $R_4$ is as defined in Claim 1.

11. A compound as claimed in Claim 1 in which Z is sulfonyl and $R^1$, $R^2$ and $R^3$ are as defined in Claim 5.

12. A compound as claimed in Claim 11 in which $R_2$ is hydrogen and $R_1$ is 5-tetrazolyl.

13. A compound as claimed in Claim 11 in which $R_1$ is

$$\overset{\displaystyle O}{\overset{\|}{-C-R_4}}.$$

14. A compound as claimed in Claim 3 in which $R_2$ and $R_3$ are as defined in Claim 5 and $R_1$ is

$$-CH_2-\overset{\displaystyle O}{\overset{\|}{C}}-R_4,$$

where $R_4$ is as defined in Claim 1.

15. A compound as claimed in Claim 3 in which $R_2$ and $R_3$ are as defined in Claim 5 and $R_1$ is 5-tetrazolylmethyl.

16. A compound as claimed in Claim 1 in which Z is sulfinyl; $R_2$ and $R_3$ are as defined in Claim 5, and $R_1$ is 5-tetrazolyl-methyl or

$$-CH_2\overset{\displaystyle O}{\overset{\|}{C}}-R_4$$

where $R_4$ is as defined in Claim 1.

17. A compound as claimed in Claim 1 in which Z is sulfonyl; $R_2$ and $R_3$ are as defined in Claim 5 and $R_1$ is as defined in Claim 16.

18. A pharmaceutical formulation for the treatment and control of asthma consisting essentially of a pharmaceutically acceptable carrier containing a compound as claimed in Claim 1.

19. A compound as claimed in Claim 1 for use in treatment and control of asthma.

**Patentansprüche**

1. 6-H-Dibenz[b,e][1,4]oxathiepin mit der Strukturformel

I 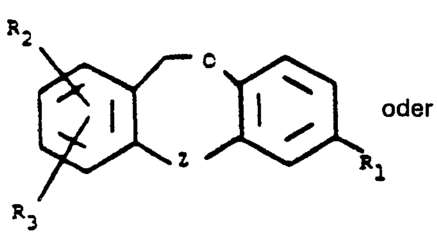 oder 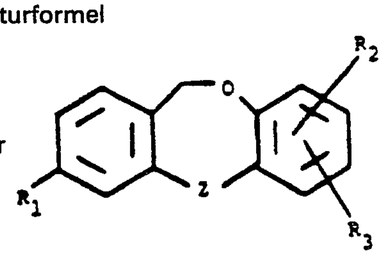 Ia

worin

Z Thia, Sufinyl oder Sulfonyl ist;

ein jedes von $R_2$ und $R_3$, die gleich oder verschieden sind, Wasserstoff, Halogen, Nitro, $C_{1-4}$-Alkyl, Amino, $C_{1-4}$-N-Alkylamino, N,N-Di($C_{1-4}$-alkyl)amino, $C_{1-4}$-Alkanoyl, Hydroxy, $C_{1-4}$-Alkoxy, $C_{1-4}$-Alkylthio, Trifluormethylthio, $C_{1-4}$-Alkylsulfinyl, $C_{1-4}$-Alkylsulfonyl oder Trifluormethyl ist;

$R_1$ 5-Tetrazolyl, 5-Tetrazolylmethyl, 3-Hydroxy-1,2,5-thiadiazol-4-yl, 4-Hydroxy-$\Delta^3$-pyrrolin-3-yl-2,5-dion oder

$$(CH_2)_n-\overset{\overset{\displaystyle O}{\|}}{C}-R_4$$

ist, worin n 0 oder eine Zahl von 1 bis 4 ist, und $R_4$ N,N-Di($C_{2-4}$-alkyl)amino($C_{1-4}$-alkoxy), $C_{1-4}$-Hydroxyalkoxy, Carboxy($C_{1-4}$-alkoxy), Amino, $C_{1-4}$-N-Alkylamino, N,N-Di($C_{1-4}$-alkyl)amino, $C_{1-4}$-Alkylsulfonylamino, Carboxy($C_{1-4}$-alkyl)amino, Carboxamido($C_{1-4}$-alkyl)amino, 2-Imino-3-methylthiazolidin, ($C_{1-4}$-Acyloxy)-($C_{1-4}$-alkoxy) oder (5-Methyl-2-oxo-1,3-dioxolan-4-yl)methoxy ist;
und Verbindungen, die pharmazeutisch annehmbare Salze davon sind.

2. Verbindung nach Anspruch 1 mit der Bezeichnung:
9-(1H-Tetrazol-5-yl)-6H-dibenz[b,e][1,4]oxathiepin-11,11-dioxid;
9-(1H-Tetrazol-5-yl)-6H-dibenz[b,e][1,4]oxathiepin-11-oxid;
2-(1H-Tetrazol-5-yl)-6H-dibenz[b,e][1,4]oxathiepin;
2-(1H-Tetrazol-5-yl)-6H-dibenz[b,e][1,4]oxathiepin-11-oxid;
2-(1H-Tetrazol-5-yl)-6H-dibenz[b,e][1,4]oxathiepin-11,11-dioxid;
Methyl-6H-dibenz[b,e][1,4]oxathiepin-9-carboxylat;
6H-Dibenzo[b,e][1,4]oxathiepin-2-carboxamid;
6H-Dibenzo[b,e][1,4]oxathiepin-2-N-methylcarboxamid;
6H-Dibenz[b,e][1,4]oxathiepin-2-N-methansulfonyl-carboxamid;
6H-Dibenz[b,e][1,4]oxathiepin-2-(3-methyl-2-thiazolidinyliden)carboxamid;
2-(3-Hydroxy-1,2,5-thiadiazol-4-yl)-6H-dibenz[b,e][1,4]oxathiepin;
2-(4-Hydroxy-$\Delta^3$-pyrrolin-3-yl-2,5-dion)-6H-dibenz[b,e][1,4]oxathiepin;
β-Hydroxyethyl-6H-dibenz[b,e][1,4]oxathiepin-2-carboxylat;
β-Dimethylaminoethyl-6H-dibenz[b,e][1,4]oxathiepin-9-carboxylat;
β-Carboxyethyl-6H-dibenz[b,e][1,4]oxathiepin-2-carboxylat;
2-(1H-Tetrazol-5-ylmethyl)-6H-dibenz[b,e][1,4]oxathiepin;
6H-Dibenz[b,e][1,4]oxathiepin-2-essigsäure-11,11-dioxid;
N-(Carboxymethyl)-6H-dibenz[b,e][1,4]oxathiepin-2-carboxamid-11,11-dioxid;
2-[1-(Pivaloyloxy)ethoxycarbonyl]-6H-dibenz[b,e][1,4]oxathiepin-11,11-dioxid;
Methyl-6H-dibenz[b,e][1,4]oxathiepin-2-carboxylat-11,11-dioxid;
Methyl-9-nitro-6H-dibenz[b,e][1,4]oxathiepin-2-carboxylat;
Methyl-9-amino-6H-dibenz[b,e][1,4]oxathiepin-2-carboxylat;
Methyl-8-amino-6H-dibenz[b,e][1,4]oxathiepin-2-carboxylat;
Methyl-8-nitro-6H-dibenz[b,e][1,4]oxathiepin-2-carboxylat;
6H-Dibenz[b,e][1,4]oxathiepin-2-essigsäure;
6H-Dibenz[b,e][1,4]oxathiepin-2-acetamid; oder
9-(1H-Tetrazol-5-yl)-6H-dibenz[b,e][1,4]oxathiepin.

3. Verbindung, wie in Anspruch 1 beansprucht, worin Z Thia ist.

4. Verbindung, wie in Anspruch 3 beansprucht, worin $R_1$ 5-Tetrazolyl oder

$$-(CH_2)_n-\overset{\overset{\displaystyle O}{\|}}{C}-R_4$$

ist, worin $R^4$ wie in Anspruch 1 definiert ist.

5. Verbindung, wie in Anspruch 4 beansprucht, worin $R_2$ Wasserstoff, Amino, Nitro oder Fluor ist und $R_3$ Wasserstoff ist; und $R_1$ 5-Tetrazolyl oder

$$-\overset{\overset{\displaystyle O}{\|}}{C}-R_4$$

ist, worin $R_4$ wie in Anspruch 1 definiert ist.

6. Verbindung, wie in Anspruch 5 beansprucht, worin $R_2$ Wasserstoff ist und $R_1$ 5-Tetrazolyl ist.

7. Verbindung, wie in Anspruch 5 beansprucht, worin $R_1$

$$\overset{\displaystyle O}{\overset{\|}{-C}}-R_4$$

ist, worin $R_4$ wie in Anspruch 1 definiert ist.

8. Verbindung, wie in Anspruch 1 beansprucht, worin Z Sulfinyl ist und $R_1$, $R_2$ und $R_3$ wie in Anspruch 5 definiert sind.

9. Verbindung, wie in Anspruch 8 beansprucht, worin $R_2$ Wasserstoff ist und $R_1$ 5-Tetrazolyl ist.

10. Verbindung, wie in Anspruch 8 beansprucht, worin $R_1$

$$\overset{\displaystyle O}{\overset{\|}{-C}}-R_4$$

ist, worin $R_4$ wie in Anspruch 1 definiert ist.

11. Verbindung, wie in Anspruch 1 beansprucht, worin Z sulfonyl ist und $R_1$, $R_2$ und $R_3$ wie in Anspruch 5 definiert sind.

12. Verbindung, wie in Anspruch 11 beansprucht, worin $R_2$ Wasserstoff ist und $R_1$ 5-Tetrazolyl ist.

13. Verbindung, wie in Anspruch 11 beansprucht, worin $R_1$

$$\overset{\displaystyle O}{\overset{\|}{-C}}-R_4$$

ist.

14. Verbindung, wie in Anspruch 3 beansprucht, worin $R_2$ und $R_3$ wie in Anspruch 5 definiert sind und $R_1$

$$-CH_2\overset{\displaystyle O}{\overset{\|}{C}}-R_4$$

ist, worin $R_4$ wie in Anspruch 1 definiert ist.

15. Verbindung, wie in Anspruch 3 beansprucht, worin $R_2$ und $R_3$ wie in Anspruch 5 definiert sind und $R_1$ 5-Tetrazolylmethyl ist.

16. Verbindung, wie in Anspruch 1 beansprucht, worin Z Sulfinyl ist, $R_2$ und $R_3$ wie in Anspruch 5 definiert sind und $R_1$ 5-Tetrazolyl-methyl oder

$$-CH_2\overset{\displaystyle O}{\overset{\|}{C}}-R_4$$

ist, worin $R_4$ wie in Anspruch 1 definiert ist.

17. Verbindung, wie in Anspruch 1 beansprucht, worin Z Sulfonyl ist; $R_2$ und $R_3$ wie in Anspruch 5 definiert sind und $R_1$ wie in Anspruch 16 definiert ist.

18. Pharmazeutische Formulierung zur Behandlung und Bekämpfung von Asthma, welche im wesentlichen aus einem pharmazeutisch annehmbaren Träger besteht, der eine Verbindung, wie in Anspruch 1 beansprucht, enthält.

19. Verbindung, wie in Anspruch 1 beansprucht, zur Verwendung bei der Behandlung und Bekämpfung von Asthma.

**Revendications**

1. 6H-dibenz(b,e)(1,4)-oxathiépine répondant à la formule développée:

I 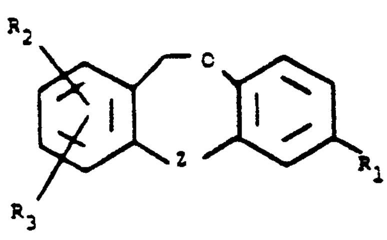 ou 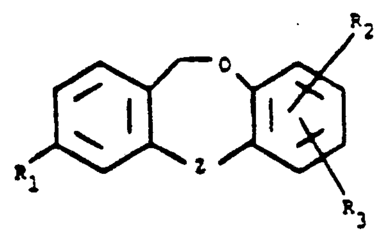 Ia

dans laquelle

Z représente le groupe thia, sulfinyl ou sulfonyle;

$R_2$ et $R_3$ qui sont identiques ou différents représentent chacun un atome d'hydrogène, d'halogène, un groupe nitro, alkyle en $C_{1-4}$, amino, N-(alkyl-en $C_{1-4}$)amino, N,N-di(alkyl- en $C_{1-4}$)amino, alcanoyle en $C_{1-4}$, hydroxyle, alcoxy en $C_{1-4}$, (alkyl- en $C_{1-4}$)thio, trifluorométhylthio, (alkyl-en $C_{1-4}$)sulfinyle, (alkyl- en $C_{1-4}$)sulfonyle ou trifluorométhyle;

$R_1$ représente un groupe 5-tétrazolyle, 5-tétrazolylméthyle, 3-hydroxy-1,2,5-thiadiazol-4-yl, 4-hydroxy-$\Delta^3$-pyrrolin-3-yl-2,5-dione ou

$$(CH_2)_n-\overset{\overset{\displaystyle O}{\|}}{C}-R_4$$

où n vaut 0 ou un nombre entier valant de 1 à 4 et $R_4$ représente un groupe N,N-di(alkyl- en $C_{1-4}$)amino-(alcoxy- en $C_{1-4}$), hydroxy(alcoxy- en $C_{1-4}$), carboxy(alcoxy- en $C_{1-4}$), amino, N-(alkyl- en $C_{1-4}$)amino, N,N-di(alkyl- en $C_{1-4}$)amino, (alkyl- en $C_{1-4}$)sulfonylamino, carboxy(alkyl-en $C_{1-4}$)amino, carboxamido(alkyl- en $C_{1-4}$)amino, 2-imino-3-méthylthiazolidine, (acyloxy en $C_{1-4}$)-(alcoxy en $C_{1-4}$) ou (5-méthyl-2-oxo-1,3-dioxolan-4-yl)méthoxy;

et les composés qui sont ses sels pharmaceutiquement acceptables.

2. Composé selon la revendication 1, ayant pour dénomination:
9-(1H-tétrazol-5-yl)-6H-dibenz(b,e)(1,4)oxathiépin-11,11-dioxyde;
9-(1H-tétrazol-5-yl)-6H-dibenz(b,e)(1,4)oxathiépin-11-oxyde;
2-(1H-tétrazol-5-yl)-6H-dibenz(b,e)(1,4)oxathiépine;
2-(1H-tétrazol-5-yl)-6H-dibenz(b,e)(1,4)oxathiépin-11-oxyde;
2-(1H-tétrazol-5-yl)-6H-dibenz(b,e)(1,4)oxathiépin-11,11-dioxyde;
6H-dibenz(b,e)(1,4)oxathiépin-9-carboxylate de méthyle;
6H-dibenzo(b,e)(1,4)oxathiépin-2-carboxamide;
6H-dibenzo(b,e)(1,4)oxathiépin-2-N-méthylcarboxamide;
6H-dibenz(b,e)(1,4)oxathiépin-2-N-méthanesulfonyl-carboxamide;
6H-dibenz(b,e)(1,4)oxathiépin-2-(3-méthyl-2-thiazolidinylidène)carboxamide;
2-(3-hydroxy-1,2,5-thiadiazol-4-yl)-6H-dibenz(b,e)(1,4)oxathiépine;
2-(4-hydroxy-$\Delta^3$-pyrrolin-3-yl-2,5-dione)-6H-dibenz(b,e)(1,4)oxathiépine;
6H-dibenz(b,e)(1,4)oxathiépin-2-carboxylate de β-hydroxyéthyle;
6H-dibenz(b,e)(1,4)oxathiépin-9-carboxylate de β-diméthylaminoéthyle;
6H-dibenz(b,e)(1,4)oxathiépin-2-carboxylate de β-carboxyéthyle;
2-(1H-tétrazol-5-yl-méthyl)-6H-dibenz(b,e)(1,4)oxathiépine;
Acide 6H-dibenz(b,e)(1,4)oxathiépin-2-acétique-11,11-dioxyde;
N-(carboxyméthyl)-6H-dibenz(b,e)(1,4)oxathiépin-2-carboxamide-11,11-dioxyde;
2-(1-pivaloyloxy)éthoxycarbonyl)-6H-dibenz(b,e)(1,4)oxathiépin-11,11-dioxyde;
6H-dibenz(b,e)(1,4)oxathiépin-2-carboxylate de méthyle-11,11-dioxyde;
9-nitro-6H-dibenz(b,e)(1,4)oxathiépin-2-carboxylate de méthyle;
9-amino-6H-dibenz(b,e)(1,4)oxathiépin-2-carboxylate de méthyle;
8-amino-6H-dibenz(b,e)(1,4)oxathiépin-2-carboxylate de méthyle;
8-nitro-6H-dibenz(b,e)(1,4)oxathiépin-2-carboxylate de méthyle;
Acide 6H-dibenz(b,e)(1,4)oxathiépin-2-acétique;
6H-dibenz(b,e)(1,4)oxathiépin-2-acétamide; ou
9-(1H-tétrazol-5-yl)-6H-dibenz(b,e)(1,4)oxathiépine.

3. Composé selon la revendication 1, dans lequel Z est le groupe thio.

4. Composé selon la revendication 3, dans lequel $R_1$ est le groupe 5-tétrazolyle ou

$$-(CH_2)_n-\overset{\overset{\displaystyle O}{\|}}{C}-R_4$$

où $R_4$ est tel que défini dans la revendication 1.

5. Composé selon la revendication 4, dans lequel $R_2$ est un atome d'hydrogène, un groupe amino, nitro ou fluoro et $R_3$ est un atome d'hydrogène; et $R_1$ est un groupe 5-tétrazolyle ou

$$-\overset{\overset{\displaystyle O}{\|}}{C}-R_4$$

où $R_4$ est tel que défini dans la revendication 1.

6. Composé selon la revendication 5, dans lequel $R_2$ est un atome d'hydrogène et $R_1$ est le groupe 5-tétrazolyle.

7. Composé selon la revendication 5, dans lequel $R_1$ est

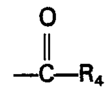

où $R_4$ est tel que défini dans la revendication 1.

8. Composé selon la revendication 1, dans lequel Z est le groupe sulfinyle et $R_1$, $R_2$ et $R_3$ sont tels que définis dans la revendication 5.

9. Composé selon la revendication 8, dans lequel $R_2$ est un atome d'hydrogène et $R_1$ est le groupe 5-tétrazolyle.

10. Composé selon la revendication 8, dans lequel $R_1$ est

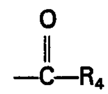

où $R_4$ est tel que défini dans la revendication 1.

11. Composé selon la revendication 1, dans lequel Z est le groupe sulfonyle et $R_1$, $R_2$ et $R_3$ sont tels que définis dans la revendication 5.

12. Composé selon la revendication 11, dans lequel $R_2$ est un atome d'hydrogène et $R_1$ est le groupe 5-tétrazolyle.

13. Composé selon la revendication 11, dans lequel $R_1$ est

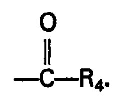

14. Composé selon la revendication 3, dans lequel $R_2$ et $R_3$ sont tels que définis dans revendication 5, et $R_1$ est

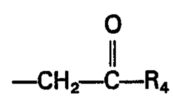

où $R_4$ est tel que défini dans la revendication 1.

15. Composé selon la revendication 3, dans lequel $R_2$ et $R_3$ sont tels que définis dans la revendication 5 et $R_1$ est le groupe 5-tétrazolylméthyle.

16. Composé selon la revendication 1, dans lequel Z est le groupe sulfinyle; $R_2$ et $R_3$ sont tels que définis dans la revendication 5 et $R_1$ est un groupe 5-tétrazolylméthyle ou

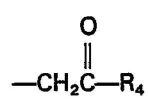

où $R_4$ est tel que défini dans la revendication 1.

17. Composé selon la revendication 1, dans lequel Z est le groupe sulfonyle; $R_2$ et $R_3$ sont tels que définis dans la revendication 5 et $R_1$ est tel que défini dans la revendication 16.

18. Formulation phamaceutique pour le traitement et la prévention de l'asthme comprenant essentiellement un véhicule pharmaceutiquement acceptable contenant un composé selon la revendication 1.

19. Composé selon la revendication 1, à utiliser dans le traitement et la prévention de l'asthme.